(19)

Europäisches
Patentamt
European
Patent Office
Office européen
des brevets

(11) **EP 4 360 813 A1**

(12) **EUROPEAN PATENT APPLICATION**
published in accordance with Art. 153(4) EPC

(43) Date of publication:
**01.05.2024 Bulletin 2024/18**

(21) Application number: **22904593.5**

(22) Date of filing: **05.12.2022**

(51) International Patent Classification (IPC):
**B25J 9/00** (2006.01)      **B25J 9/16** (2006.01)
**B25J 13/00** (2006.01)      **B25J 13/06** (2006.01)
**B25J 13/08** (2006.01)

(52) Cooperative Patent Classification (CPC):
**A61F 2/605; A61F 2/70; A61H 1/0237; A61H 3/00;
A63B 21/00178; A63B 21/00181; A63B 21/4011;**
A61F 2002/704; A61H 2003/007; A61H 2201/165;
A61H 2201/5007; A61H 2201/5061;
A61H 2201/5069; A61H 2201/5084; A61H 2230/625

(86) International application number:
**PCT/KR2022/019609**

(87) International publication number:
**WO 2023/106763 (15.06.2023 Gazette 2023/24)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **06.12.2021 KR 20210173188
19.08.2022 KR 20220104187**

(71) Applicant: **Samsung Electronics Co., Ltd.
Suwon-si, Gyeonggi-do 16677 (KR)**

(72) Inventors:
• **HWANG, Jungsik**
  **Suwon-si Gyeonggi-do 16677 (KR)**
• **LIM, Bokman**
  **Suwon-si Gyeonggi-do 16677 (KR)**
• **KIM, Sungcheol**
  **Suwon-si Gyeonggi-do 16677 (KR)**

(74) Representative: **Gulde & Partner
Patent- und Rechtsanwaltskanzlei mbB
Wallstraße 58/59
10179 Berlin (DE)**

(54) **METHOD FOR GENERATING MOTION CONTROL MODEL FOR WEARABLE APPARATUS, AND ELECTRONIC APPARATUS PERFORMING SAID METHOD**

(57) An electronic apparatus according to an embodiment may: provide a user with at least one of target motion information regarding a target motion of a wearable apparatus according to a target time and default torque information mapped to the information regarding the target motion and output by the wearable apparatus; receive, from the user, an input for modifying at least a portion of the default torque information; generate modified torque information on the basis of the default torque information and the input, and generate, on the basis of the information regarding the target motion and the modified torque information, a motion control model for controlling the wearable apparatus.

Start

710

Load target motion information and basic torque information

720

Provide user with target motion information and basic torque information

730

Receive, from user, input for modifying at least portion of basic torque information

740

Generate modified torque information based on basic torque information and input

750

Generate motion control model for controlling wearable device based on target motion information and modified torque information

760

Control operation of wearable device based on motion control model

End

FIG. 7

**Description**

Technical Field

Certain example embodiments relate to a technology for generating a motion control model that controls a wearable device.

Background Art

A change into aging societies has contributed to a growing number of people who experience inconvenience and pain from reduced muscular strength or joint problems due to aging. Thus, there is a growing interest in walking assist devices that enable elderly users or patients with reduced muscular strength or joint problems to walk with less effort.

Disclosure of the Invention

Technical Goals

According to an example embodiment, an electronic device may include a communication module, comprising communication circuitry, configured to exchange data with an external device, and at least one processor configured to control the electronic device, wherein the at least one processor may be configured to provide a user of the electronic device with at least one of target motion information of a wearable device according to a target period or basic torque information mapped to the target motion information and output by the wearable device, receive an input for modifying at least a portion of the basic torque information from the user, generate modified torque information based on the basic torque information and the input, and generate a motion control model for controlling the wearable device based on the target motion information and the modified torque information, wherein an operation of the wearable device may be controlled based on the motion control model.

According to an example embodiment, a method of generating a motion control model for controlling a wearable device may be provided, to be performed by an electronic device, and may include providing a user of the electronic device with at least one of target motion information of the wearable device according to a target period or basic torque information mapped to the target motion information and output by the wearable device, receiving an input for modifying at least a portion of the basic torque information from the user, generating modified torque information based on the basic torque information and the input, and generating a motion control model for controlling the wearable device based on the target motion information and the modified torque information, wherein an operation of the wearable device may be controlled based on the motion control model.

According to an example embodiment, an electronic device may include a communication module, comprising communication circuitry, configured to exchange data with an external device, and at least one processor configured to control the electronic device, wherein the at least one processor may be configured to receive target motion information of the wearable device during a target period from the wearable device in a state in which a user is wearing the wearable device, receive first torque information with respect to a first time within the target period from the user, generate basic torque information based on the first torque information, and generate a motion control model for controlling the wearable device based on the target motion information and the basic torque information, wherein an operation of the wearable device may be controlled based on the motion control model.

Brief Description of Drawings

FIG. 1 is a diagram illustrating a configuration of a system for providing a user with a workout program according to an example embodiment.
FIG. 2 is a block diagram of an electronic device in a network environment according to an example embodiment.
FIGS. 3A, 3B, 3C, and 3D are diagrams illustrating a wearable device according to an example embodiment.
FIG. 4 is a diagram illustrating a wearable device communicating with an electronic device according to an example embodiment.
FIGS. 5 and 6 are diagrams illustrating a torque output method of a wearable device according to an example embodiment.
FIG. 7 is a flowchart illustrating a method of generating a motion control model for a wearable device according to an example embodiment.
FIG. 8 illustrates target motion information of a wearable device and basic torque information mapped to the target motion information according to an example embodiment.
FIG. 9 is a flowchart illustrating a method of generating modified torque information based on an input received from a user according to an example embodiment.
FIG. 10 illustrates modified torque information generated based on basic torque information and an input of a user according to an example embodiment.
FIG. 11 is a flowchart illustrating a method of loading target motion information and basic torque information according to an example embodiment.
FIG. 12 is a flowchart illustrating a method of receiving target motion information using a wearable device according to an example embodiment.
FIG. 13 illustrates a user interface (UI) screen for receiving torque information with respect to target motion information from a user according to an example embodiment.

FIG. 14 is a flowchart illustrating a method of generating basic torque information based on first torque information and second torque information according to an example embodiment.

FIG. 15 illustrates a UI screen for generating modified torque information based on loaded basic torque information according to an example embodiment.

FIG. 16 is a diagram illustrating a configuration of a server according to an example embodiment.

Best Mode for Carrying Out the Invention

Hereinafter, various example embodiments of the present disclosure will be described with reference to the accompanying drawings. However, this is not intended to limit the present disclosure to specific embodiments, and it should be understood that various modifications, equivalents, and/or alternatives of the embodiments of the present disclosure are included.

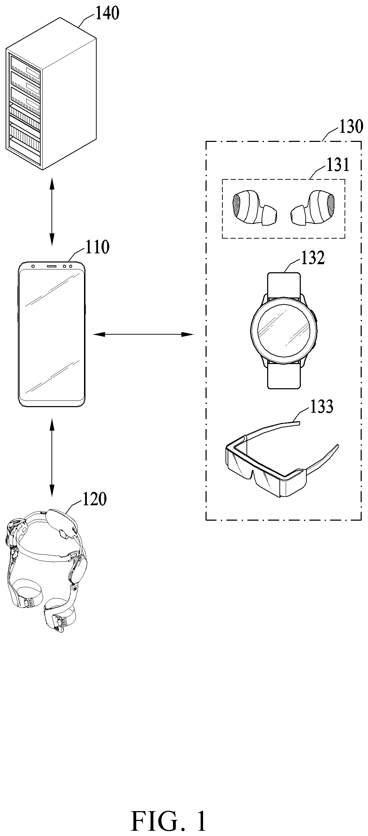

FIG. 1 is a diagram illustrating a configuration of a system for providing a user with a workout program according to an example embodiment.

According to an example embodiment, a system for providing a user with a workout program may include an electronic device 110, a wearable device 120, an additional device 130, and a server 140.

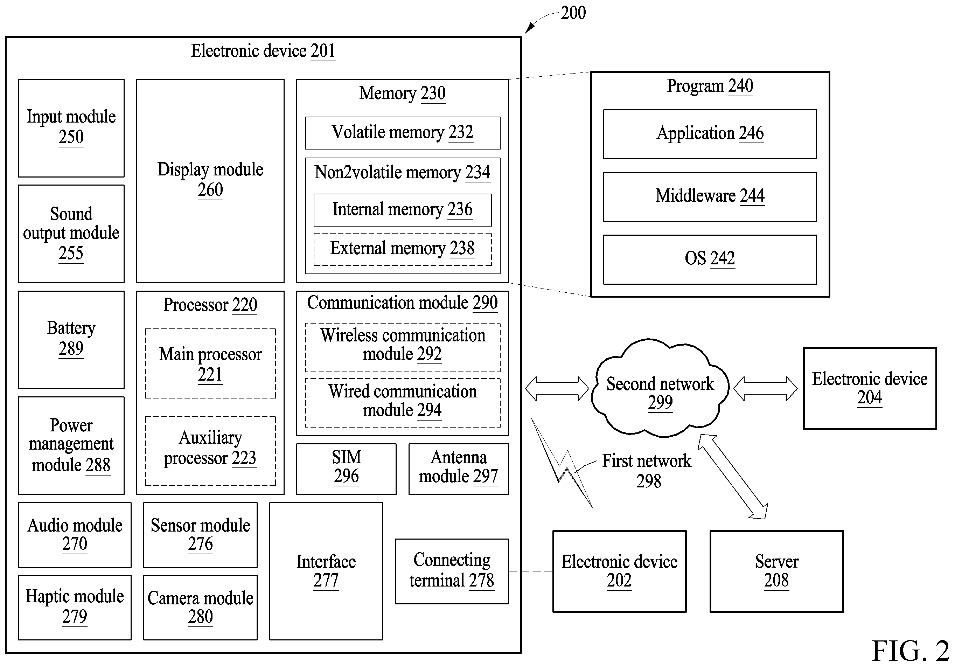

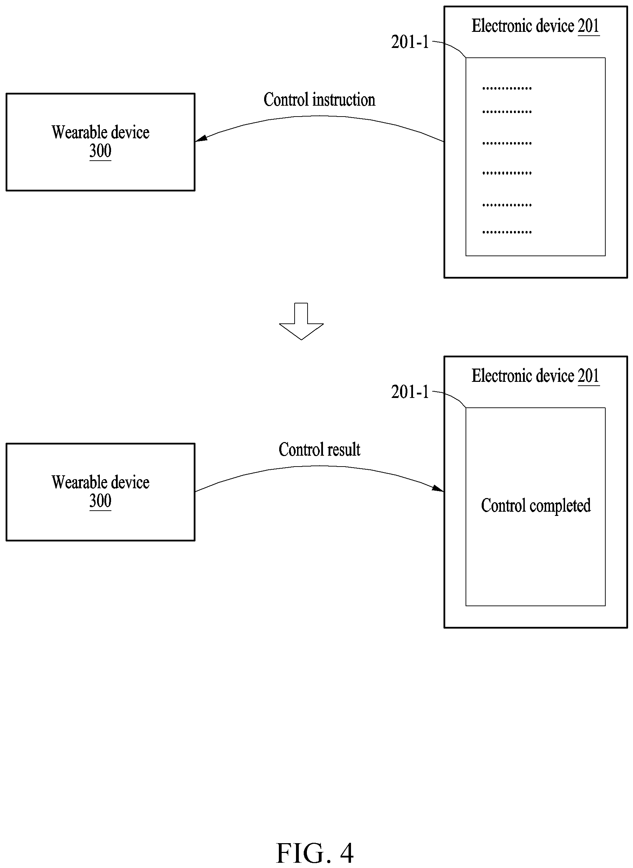

According to an example embodiment, the electronic device 110 may be a user terminal that may be connected, directly or indirectly, to the wearable device 120 using short-range wireless communication. For example, the electronic device 110 may transmit a control signal for controlling the wearable device 120 to the wearable device 120. The electronic device 110 will be described in detail below with reference to FIG. 2, and the transmission of a control signal will be described in detail below with reference to FIG. 4.

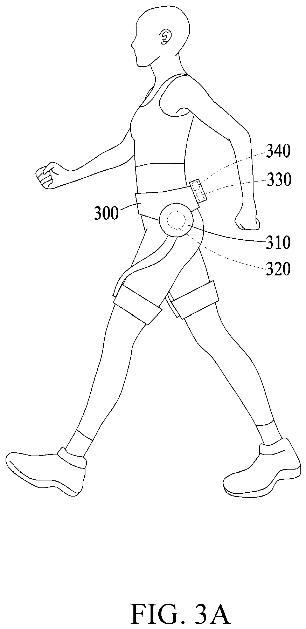

According to an example embodiment, the wearable device 120 may provide a user wearing the wearable device 120 with an assistance force for assisting a gait or a workout or a resistance force for impeding a gait. The resistance force may be provided to the user to assist the user in doing a workout. The values of various control parameters used in the wearable device 120 may be controlled to control the assistance force or the resistance force output by the wearable device 120. The structure and driving method of the wearable device 120 will be described in detail below with reference to FIGS. 3A, 3B, 3C, 3D, 4, 5, and 6.

According to an example embodiment, the electronic device 110 may be connected to the additional device 130 (e.g., wireless earphones 131, a smart watch 132, or smart glasses 133) using short-range wireless communication. For example, the electronic device 110 may output information indicating the state of the electronic device 110 or the state of the wearable device 120 to the user through the additional device 130. For example, feedback information with respect to a walking state of the user wearing the wearable device 120 may be output through a haptic device, a speaker device, and a display device of the additional device 130.

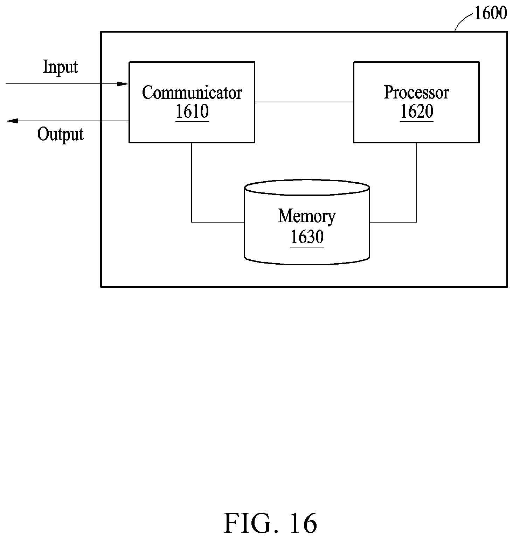

According to an example embodiment, the electronic device 110 may be connected to the server 140 using short-range wireless communication or cellular communication. For example, the server 140 may include a database in which information about a plurality of workout programs to be provided to a user through the wearable device 120 is stored. A workout program may be based on a motion control model for controlling the wearable device 120 to provide the user with a torque appropriate for a target motion of the user. For example, the server 140 may manage a user account of the user of the electronic device 110 or the wearable device 120. The server 140 may store and manage a workout program performed by the user and a result of performance with respect to the workout program in link with the user account. An example of the configuration of the server 140 will be described in detail below with reference to FIG. 16.

According to an example embodiment, the system may provide the user with a workout program for a motion desired by the user. For example, the motion desired by the user may be a standardized in-place workout such as a squat, a lunge, or a kickback. For example, the motion desired by the user may be a workout arbitrarily made by the user.

According to an example embodiment, when the user wearing the wearable device 120 performs a motion, the workout program may provide the user with a force (or a torque) preset to correspond to the motion through the wearable device 120. For example, the force provided to the user may be an assistance force. For example, the force provided to the user may be a resistance force. The output timing and magnitude of the force provided to the user by the wearable device 120 may be controlled by the user modifying the existing workout program or authoring a new workout program.

According to an example embodiment, the user may generate a motion control model for a workout program to control the wearable device 120 through the electronic device 110. For example, the user may generate the motion control model by determining values of control parameters related to a torque output during a target motion of the wearable device 120. For example, the control parameters may include parameters for adjusting at least one of a magnitude of a torque to be output through the wearable device 120, a direction of the torque, a timing of the torque, an offset angle between joint angles of the wearable device 120, or a sensitivity of a state factor with respect to the joint angles.

According to an example embodiment, considering difficulties of a user in designating the values of the control parameters one by one when generating the motion control model, a method of obtaining from the user the direction and magnitude of a force desired by the user to be output with respect to a predetermined motion among all motions and automatically generating values of control parameters based on the obtained information may be used in the process of generating the motion control model. For example, the user may generate a motion control model for a desired motion through the electronic device 110, and the electronic device 110 may provide the user with a workout program by controlling the wearable device 120 through the motion control model.

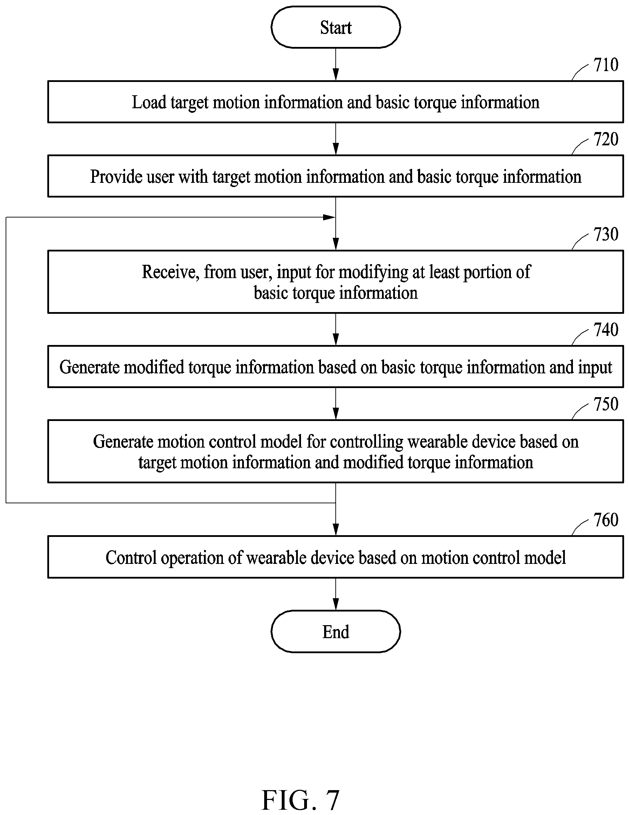

A method of generating the motion control model will be described in detail below with reference to FIGS. 7 to 15.

FIG. 2 is a block diagram of an electronic device in a network environment according to an example embodiment.

FIG. 2 is a block diagram of an electronic device 201 (e.g., the electronic device 110 of FIG. 1) in a network environment 200 according to an example embodiment. Referring to FIG. 2, the electronic device 201 in the network environment 200 may communicate with an electronic device 202 via a first network 298 (e.g., a short-range wireless communication network), or communicate with at least one of an electronic device 204 or a server 208 via a second network 299 (e.g., a long-range wireless communication network). According to an example embodiment, the electronic device 201 may communicate with the electronic device 204 via the server 208. According to an example embodiment, the electronic device 201 may include a processor 220, a memory 230, an input module 250, a sound output module 255, a display module 260, an audio module 270, a sensor module 276, an interface 277, a connecting terminal 278, a haptic module 279, a camera module 280, a power management module 288, a battery 289, a communication module 290, a subscriber identification module (SIM) 296, or an antenna module 297. In some embodiments, at least one (e.g., the connecting terminal 278) of the above components may be omitted from the electronic device 201, or one or more other components may be added in the electronic device 201. In some embodiments, some (e.g., the sensor module 276, the camera module 280, or the antenna module 297) of the components may be integrated as a single component (e.g., the display module 260).

The processor 220 may execute, for example, software (e.g., a program 240) to control at least one other component (e.g., a hardware or software component) of the electronic device 201 connected to the processor 220, and may perform various data processing or computation. According to an example embodiment, as at least a portion of data processing or computation, the processor 220 may store a command or data received from another component (e.g., the sensor module 276 or the communication module 290) in a volatile memory 232, process the command or the data stored in the volatile memory 232, and store resulting data in a non-volatile memory 234. According to an example embodiment, the processor 220 may include a main processor 221 (e.g., a central processing unit (CPU) or an application processor (AP)) or an auxiliary processor 223 (e.g., a graphics processing unit (GPU), a neural processing unit (NPU), an image signal processor (ISP), a sensor hub processor, or a communication processor (CP)) that is operable independently of, or in conjunction with the main processor 221. For example, when the electronic device 201 includes the main processor 221 and the auxiliary processor 223, the auxiliary processor 223 may be adapted to consume less power than the main processor 221 or to be specific to a specified function. The auxiliary processor 223 may be implemented separately from the main processor 221 or as a portion of the main processor 221.

The auxiliary processor 223 may control at least some of functions or states related to at least one (e.g., the display module 260, the sensor module 276, or the communication module 290) of the components of the electronic device 201, instead of the main processor 221 while the main processor 221 is in an inactive (e.g., sleep) state or along with the main processor 221 while the main processor 221 is in an active state (e.g., executing an application). According to an example embodiment, the auxiliary processor 223 (e.g., an ISP or a CP) may be implemented as a portion of another component (e.g., the camera module 280 or the communication module 290) that is functionally related to the auxiliary processor 223. According to an example embodiment, the auxiliary processor 223 (e.g., an NPU) may include a hardware structure specified for artificial intelligence (AI) model processing. An artificial intelligence model may be generated by machine learning. Such learning may be performed by, for example, the electronic device 201 in which an artificial intelligence model is executed, or performed via a separate server (e.g., the server 208). Learning algorithms may include, but are not limited to, for example, supervised learning, unsupervised learning, semi-supervised learning, or reinforcement learning. The artificial intelligence model may include a plurality of artificial neural network layers. An artificial neural network may include, for example, a deep neural network (DNN), a convolutional neural network (CNN), a recurrent neural network (RNN), a restricted Boltzmann machine (RBM), a deep belief network (DBN), and a bidirectional recurrent deep neural network (BRDNN), a deep Q-network, or a combination of two or more thereof, but is not limited thereto. The AI model may additionally or alternatively include a software structure other than the hardware structure.

The memory 230 may store various data used by at least one component (e.g., the processor 220 or the sensor module 276) of the electronic device 201. The various data may include, for example, software (e.g., the program 240) and input data or output data for a command related thereto. The memory 230 may include the volatile memory 232 or the non-volatile memory 234.

The program 240 may be stored as software in the memory 230, and may include, for example, an operating system (OS) 242, middleware 244, or an application 246.

The input module 250 may receive a command or data to be used by another component (e.g., the processor 220) of the electronic device 201, from the outside (e.g., a user) of the electronic device 201. The input module 250 may include, for example, a microphone, a mouse, a keyboard, a key (e.g., a button), or a digital pen (e.g., a stylus pen).

The sound output module 255 may output a sound signal to the outside of the electronic device 201. The sound output module 255 may include, for example, a speaker or a receiver. The speaker may be used for general purposes, such as playing multimedia or playing record. The receiver may be used to receive an incoming call. According to an example embodiment, the receiver may be implemented separately from the speaker or as a portion of the speaker.

The display module 260 may visually provide information to the outside (e.g., a user) of the electronic device 201. The display module 260 may include, for example, a control circuit for controlling a display, a hologram device, or a projector and control circuitry to control a corresponding one of the display, the hologram device, and the projector. According to an example embodiment, the display module 260 may include a touch sensor adapted to sense a touch, or a pressure sensor adapted to measure an intensity of a force incurred by the touch.

The audio module 270 may convert a sound into an electrical signal or vice versa. According to an example embodiment, the audio module 270 may obtain the sound via the input module 250 or output the sound via the sound output module 255 or an external electronic device (e.g., an electronic device 202 such as a speaker or a headphone) directly or wirelessly connected to the electronic device 201.

The sensor module 276 may detect an operational state (e.g., power or temperature) of the electronic device 201 or an environmental state (e.g., a state of a user) external to the electronic device 201, and generate an electrical signal or data value corresponding to the detected state. According to an example embodiment, the sensor module 276 may include, for example, a gesture sensor, a gyro sensor, an atmospheric pressure sensor, a magnetic sensor, an acceleration sensor, a grip sensor, a proximity sensor, a color sensor, an infrared (IR) sensor, a biometric sensor, a temperature sensor, a humidity sensor, or an illuminance sensor.

The interface 277 may support one or more specified protocols to be used for the electronic device 201 to be coupled with the external electronic device (e.g., the electronic device 202) directly (e.g., wiredly) or wirelessly. According to an example embodiment, the interface 277 may include, for example, a high-definition multimedia interface (HDMI), a universal serial bus (USB) interface, a secure digital (SD) card interface, or an audio interface.

The connecting terminal 278 may include a connector via which the electronic device 201 may be physically connected to an external electronic device (e.g., the electronic device 202). According to an example embodiment, the connecting terminal 278 may include, for example, an HDMI connector, a USB connector, an SD card connector, or an audio connector (e.g., a headphone connector).

The haptic module 279 may convert an electrical signal into a mechanical stimulus (e.g., a vibration or a movement) or an electrical stimulus which may be recognized by a user via his or her tactile sensation or kinesthetic sensation. According to an example embodiment, the haptic module 279 may include, for example, a motor, a piezoelectric element, or an electric stimulator.

The camera module 280, comprising at least one camera, may take a still image and a video. According to an example embodiment, the camera module 280 may include one or more lenses, image sensors, ISPs, or flashes.

The power management module 288 may manage power supplied to the electronic device 201. According to an example embodiment, the power management module 288 may be implemented as, for example, at least a portion of a power management integrated circuit (PMIC).

The battery 289 may supply power to at least one component of the electronic device 201. According to an example embodiment, the battery 289 may include, for example, a primary cell which is not rechargeable, a secondary cell which is rechargeable, or a fuel cell.

The communication module 290, comprising communication circuitry, may support establishing a direct (e.g., wired) communication channel or a wireless communication channel between the electronic device 201 and the external electronic device (e.g., the electronic device 202, the electronic device 204, or the server 208) and performing communication via the established communication channel. The communication module 290 may include one or more communication processors that operate independently of the processor 220 (e.g., an application processor) and support direct (e.g., wired) communication or wireless communication. According to an example embodiment, the communication module 290 may include a wireless communication module 292 (e.g., a cellular communication module, a short-range wireless communication module, or a global navigation satellite system (GNSS) communication module) or a wired communication module 294 (e.g., a local area network (LAN) communication module, or a power line communication (PLC) module). A corresponding one of these communication modules may communicate with the external electronic device 204 via the first network 298 (e.g., a short-range communication network, such as Bluetooth™, wireless-fidelity (Wi-Fi) direct, or infrared data association (IrDA)) or the second network 299 (e.g., a long-range communication network, such as a legacy cellular network, a 5G network, a next-generation communication network, the Internet, or a computer network (e.g., a LAN or a wide area network (WAN))). These various types of communication modules may be implemented as a single component (e.g., a single chip), or may be implemented as multi components (e.g., multi chips) separate from each other. The wireless communication module 292 may identify and authenticate the electronic device 201 in a communication network, such as the first network 298 or the second network 299, using subscriber information (e.g., international mobile subscriber identity (IMSI)) stored in the SIM 296.

The wireless communication module 292, comprising communication circuitry, may support a 5G network after a 4G network, and a next-generation communication technology, e.g., a new radio (NR) access technology. The NR access technology may support enhanced mobile broadband (eMBB), massive machine type communications (mMTC), or ultra-reliable and low-latency communications (URLLC). The wireless communication module 292 may support a high-frequency band (e.g., a mmWave band) to achieve, e.g., a high data transmission rate. The wireless communication module 292 may support various technologies for securing performance on a high-frequency band, such as, e.g., beam-forming, massive multiple-input and multiple-output (MIMO), full dimensional MIMO (FD-MIMO), an array antenna, analog beam-forming, or a large scale antenna. The wireless communication module 292 may support various requirements specified in the electronic device 201, an external electronic device (e.g., the electronic device 204), or a network system (e.g., the second network 299). According to an example embodiment, the wireless communication module 292 may support a peak data rate (e.g., 20 Gbps or more) for implementing eMBB, loss coverage (e.g., 164 dB or less) for implementing mMTC, or U-plane latency (e.g., 0.5 ms or less for each of downlink (DL) and uplink (UL), or a round trip of 1 ms or less) for implementing URLLC.

The antenna module 297 may transmit or receive a signal or power to or from the outside (e.g., the external electronic device) of the electronic device 201. According to an example embodiment, the antenna module 297 may include an antenna including a radiating element including a conductive material or a conductive pattern formed in or on a substrate (e.g., a printed circuit board (PCB)). According to an example embodiment, the antenna module 297 may include a plurality of antennas (e.g., array antennas). In such a case, at least one antenna appropriate for a communication scheme used in a communication network, such as the first network 298 or the second network 299, may be selected by, for example, the communication module 290 from the plurality of antennas. The signal or the power may be transmitted or received between the communication module 290 and the external electronic device via the at least one selected antenna. According to an example embodiment, another component (e.g., a radio frequency integrated circuit (RFIC)) other than the radiating element may be additionally formed as a portion of the antenna module 297 comprising at least one antenna.

According to an example embodiment, the antenna module 297 may form a mmWave antenna module. For example, the mmWave antenna module may include a PCB, an RFIC disposed on a first surface (e.g., a bottom surface) of the PCB or adjacent to the first surface and capable of supporting a designated a high-frequency band (e.g., the mmWave band), and a plurality of antennas (e.g., array antennas) disposed on a second surface (e.g., a top or a side surface) of the PCB, or adjacent to the second surface and capable of transmitting or receiving signals in the designated high-frequency band.

At least some of the above-described components may be coupled mutually and communicate signals (e.g., commands or data) therebetween via an inter-peripheral communication scheme (e.g., a bus, general purpose input and output (GPIO), serial peripheral interface (SPI), or mobile industry processor interface (MIPI)).

According to an example embodiment, commands or data may be transmitted or received between the electronic device 201 and the external electronic device 204 via the server 208 coupled with the second network 299. Each of the external electronic devices 202 and 204 may be a device of the same type as or a different type from the electronic device 201. According to an example embodiment, all or some of operations to be executed by the electronic device 201 may be executed at one or more of the external electronic devices 202, 204, and 208. For example, if the electronic device 201 needs to perform a function or a service automatically, or in response to a request from a user or another device, the electronic device 201, instead of, or in addition to, executing the function or the service, may request one or more external electronic devices to perform at least portion of the function or the service. The one or more external electronic devices receiving the request may perform the at least portion of the function or the service requested, or an additional function or an additional service related to the request, and may transfer an outcome of the performing to the electronic device 201. The electronic device 201 may provide the outcome, with or without further processing of the outcome, as at least portion of a reply to the request. To that end, a cloud computing, distributed computing, mobile edge computing (MEC), or client-server computing technology may be used, for example. The electronic device 201 may provide ultra low-latency services using, e.g., distributed computing or mobile edge computing. In an example embodiment, the external electronic device 204 may include an Internet-of-things (IoT) device. The server 208 may be an intelligent server using machine learning and/or a neural network. According to an example embodiment, the external electronic device 204 or the server 208 may be included in the second network 299. The electronic device 201 may be applied to intelligent services (e.g., smart home, smart city, smart car, or healthcare) based on 5G communication technology or IoT-related technology.

The electronic device according to an example embodiment may be one of various types of electronic devices. The electronic device may include, for example, a portable communication device (e.g., a smart phone), a computer device, a portable multimedia device, a portable medical device, a camera, a wearable device, or a home appliance device. According to an example embodiment of the disclosure, the electronic device is not limited to those described above.

It should be appreciated that various embodiments of the present disclosure and the terms used therein are not intended to limit the technological features set forth herein to particular embodiments and include various changes, equivalents, or replacements for a corresponding embodiment. In connection with the description of the drawings, like reference numerals may be used for similar or related components. It is to be understood that a singular form of a noun corresponding to an item may include one or more of the things, unless the relevant context clearly indicates otherwise. As used herein, "A or B", "at least one of A and B", "at least one of A or B", "A, B or C", "at least one of A, B and C", and "at least one of A, B, or C", each of which may include any one of the items listed together in the corresponding one of the phrases, or all possible combinations thereof. Terms such as "first", "second", or "first" or "second" may simply be used to distinguish the component from other components in question, and do not limit the components in other aspects (e.g., importance or order). It is to be understood that if an element (e.g., a first element) is referred to, with or without the term "operatively" or "communicatively", as "coupled with," "coupled to," "connected with," or "connected to" another element (e.g., a second element), it means that the element may be coupled with the other element directly (e.g., by wire), wirelessly, or via at least a third element.

As used in connection with various embodiments of the disclosure, the term "module" may include a unit implemented in hardware, software, or firmware, and may interchangeably be used with other terms, for example, "logic," "logic block," "part," or "circuitry". A module may be a single integral component, or a minimum unit or part thereof, adapted to perform one or more functions. For example, according to an example embodiment, the module may be implemented in a form of an application-specific integrated circuit (ASIC). Thus, each "module" herein may comprise circuitry.

Various embodiments as set forth herein may be implemented as software (e.g., the program 240) including one or more instructions that are stored in a storage medium (e.g., which may include an internal memory 236 and/or an external memory 238) that is readable by a machine (e.g., the electronic device 201). For example, a processor (e.g., the processor 220) of the machine (e.g., the electronic device 201) may invoke at least one of the one or more instructions stored in the storage medium, and execute it. This allows the machine to be operated to perform at least one function according to the at least one instruction invoked. The one or more instructions may include a code generated by a compiler or a code executable by an interpreter. The machine-readable storage medium may be provided in the form of a non-transitory storage medium. Here, the term "non-transitory" simply means that the storage medium is a tangible device, and does not include a signal (e.g., an electromagnetic wave), but this term does not differentiate between where data is semi-permanently stored in the storage medium and where the data is temporarily stored in the storage medium.

According to an example embodiment, a method according to various embodiments of the disclosure may be included and provided in a computer program product. The computer program product may be traded as a product between a seller and a buyer. The computer program product may be distributed in the form of a machine-readable storage medium (e.g., compact disc read only memory (CD-ROM)), or be distributed (e.g., downloaded or uploaded) online via an application store (e.g., PlayStore™), or between two user devices (e.g., smart phones) directly. If distributed online, at least portion of the computer program product may be temporarily generated or at least temporarily stored in the machine-readable storage medium, such as memory of the manufacturer's server, a server of the application store, or a relay server.

According to an example embodiment, each component (e.g., a module or a program) of the above-described components may include a single entity or multiple entities, and some of the multiple entities may be separately disposed in different components. According to an example embodiment, one or more of the above-described components may be omitted, or one or more other components may be added. Alternatively or additionally, a plurality of components (e.g., modules or programs) may be integrated into a single component. In such a case, according to various embodiments, the integrated component may still perform one or more functions of each of the plurality of components in the same or similar manner as they are performed by a corresponding one of the plurality of components before the integration. According to various embodiments, operations performed by the module, the program, or another component may be carried out sequentially, in parallel, repeatedly, or heuristically, or one or more of the operations may be executed in a different order or omitted, or one or more other operations may be added.

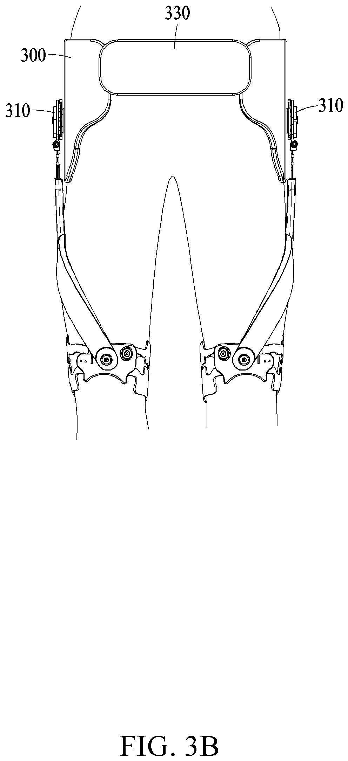

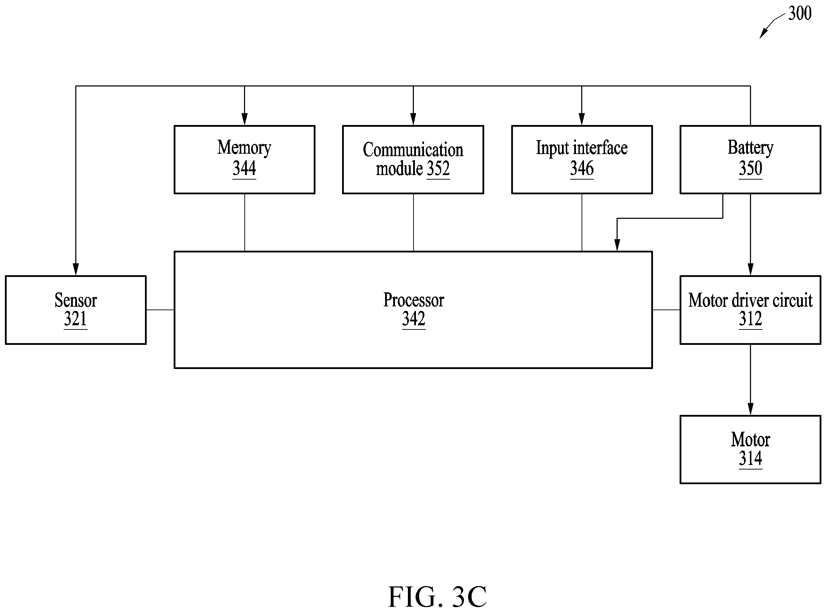

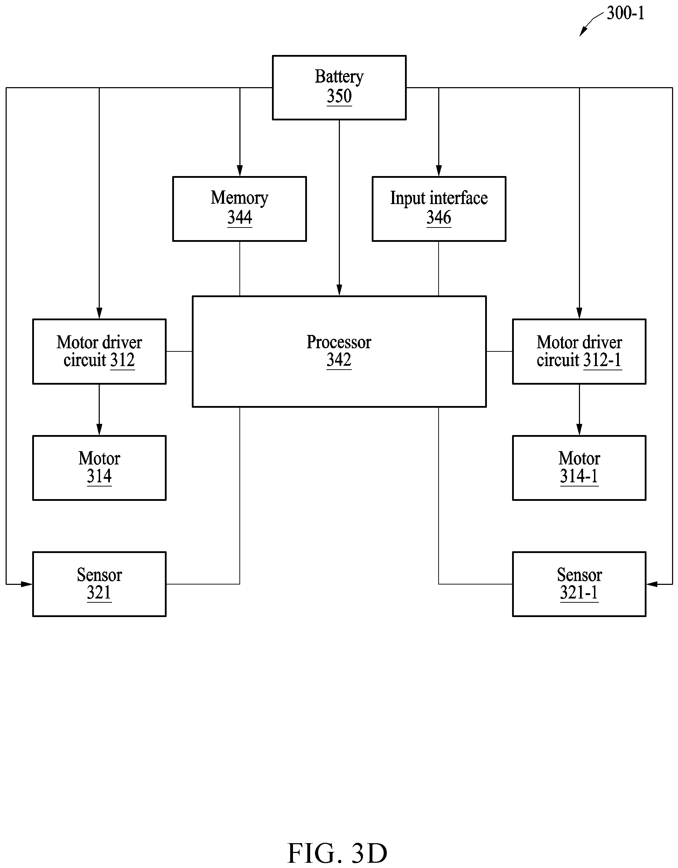

FIGS. 3A, 3B, 3C, and 3D are diagrams illustrating a wearable device according to an example embodiment.

Referring to FIGS. 3A, 3B, 3C, and 3D, a wearable device 300 (e.g., the wearable device 120 of FIG. 1) may be worn by a user to assist a gait (e.g., walking) of the user. For example, the wearable device 300 may be a device for assisting a gait (e.g., walking) of a user. Further, the wearable device 300 may be a workout device that provides a workout function by assisting a motion (e.g., a gait or a workout) of the user and providing the user with a resistance force. For example, the resistance force provided to the user may be a force actively applied to the user, such as a force output by a device such as a motor. Alternatively, the resistance force may not be a force actively applied to the user, but may be a force that impedes a motion of the user, such as a frictional force. The resistance force may also be referred to as a workout load.

Although FIGS. 3A, 3B, 3C, and 3D illustrate a hip-type wearable device 300, the type of the wearable device is not limited thereto. The wearable device may be a type that supports the entire lower limbs or a type that supports a portion of the lower limbs. In addition, the wearable device may be one of a type that supports a portion of the lower limbs, a type that supports up to the knees, a type that supports up to the ankles, and a type that supports the entire body.

The embodiments described with reference to FIGS. 3A, 3B, 3C, and 3D may apply to a hip-type wearable device, but are not limited thereto, and may all apply to various types of wearable devices.

According to an example embodiment, the wearable device 300 may include a driver 310, a sensor unit 320 comprising at least one sensor, an inertial measurement unit (IMU) 330 comprising at least one sensor, a controller 340, a battery 350, and a communication module 352. For example, the IMU 330 and the controller 340 may be disposed in a main frame of the wearable device 300. For example, the IMU 330 and the controller 340 (comprising processing circuitry) may be included in a housing that is formed in (or attached to) the outside of the main frame of the wearable device 300.

The driver 310 may include a motor 314 and a motor driver circuit 312 for driving the motor 314. The sensor unit 320 may include at least one sensor 321. The controller 340 may include a processor 342, a memory 344, and an input interface 346. Although the wearable device 300 is illustrated in FIG. 3C as including one sensor 321, one motor driver circuit 312, and one motor 314, this may be provided merely as an example, and a wearable device 300-1 may include a plurality of sensors 321 and 321-1, a plurality of motor driver circuits 312 and 312-1, and a plurality of motors 314 and 314-1 according to another example as illustrated in FIG. 3D. Also, according to implementation, the wearable device 300 may include a plurality of processors. The number of motor driver circuits, the number of motors, or the number of processors may vary depending on a body part on which the wearable device 300 is worn.

The following description of the sensor 321, the motor driver circuit 312, and the motor 314 may also apply to the sensor 321-1, the motor driver circuit 312-1, and the motor 314-1 illustrated in FIG. 3D.

The driver 310 may drive a hip joint of a user. For example, the driver 310 may be positioned on the right hip portion and/or the left hip portion of the user. The driver 310 may be additionally positioned on the knee portions and the ankle portions of the user. The driver 310 may include the motor 314 for generating a rotational torque and the motor driver circuit 312 for driving the motor 314.

The sensor unit 320 may measure the angles of the hip joints of the user during a gait. Information on the angles of the hip joints sensed by the sensor unit 320 may include the angle of the right hip joint, the angle of the left hip joint, the difference between the angles of both hip joints, and the hip joint motion direction. For example, the sensor 321 may be positioned in the driver 310. According to the position of the sensor 321, the sensor unit 320 may additionally measure the angles of the knees and the angles of the ankles of the user. The sensor 321 may be an encoder. The information on the angles of the joints measured by the sensor unit 320 may be transmitted to the controller 340.

According to an example embodiment, the sensor unit 320 may include a potentiometer. The potentiometer may sense an R-axis joint angle, an L-axis joint angle, an R-axis joint angular velocity, and an L-axis joint angular velocity according to a gait motion of the user. In this example, the R and L axes may be reference axes for the right leg and the left leg of the user, respectively. For example, the R and L axes may be set to be vertical to the ground and set such that a front side of a body of a person has a negative value and a rear side of the body has a positive value.

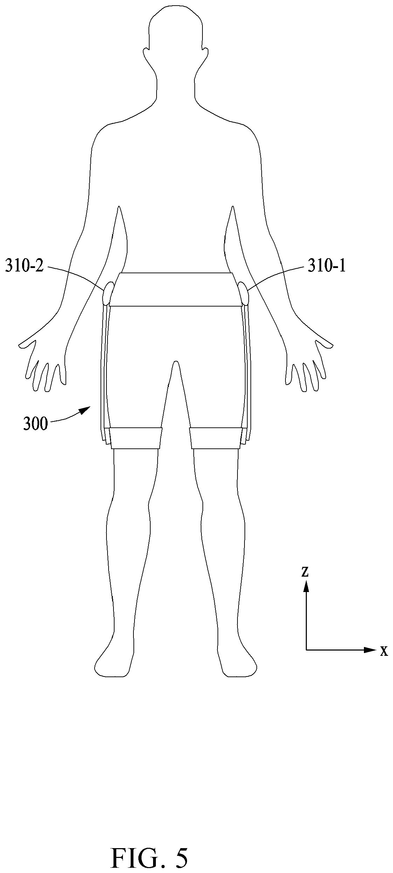

The IMU 330 may measure acceleration information and pose information during a gait. For example, the IMU 330 may sense X-axis, Y-axis, and Z-axis accelerations and X-axis, Y-axis, and Z-axis angular velocities according to the gait motion of the user (e.g., see x, y and z axes in Figs. 5-6). The acceleration information and pose information measured by the IMU 330 may be transmitted to the controller 340.

In addition to the sensor unit 320 and the IMU 330 described above, the wearable device 300 may include a sensor (e.g., an electromyogram (EMG) sensor) configured to sense a change in a quantity of motion of the user or a change in a biosignal according to a gait motion.

The controller 340, comprising processing circuitry, may control an overall operation of the wearable device 300. For example, the controller 340 may receive the information sensed by each of the sensor unit 320 and the IMU 330. The information sensed by the IMU 330 may include acceleration information and pose information, and the information sensed by the sensor unit 320 may include the angle of the right hip joint, the angle of the left hip joint, the difference between the angles of the two hip joints, and the hip joint motion direction. According to an example embodiment, the controller 340 may calculate the difference between the angles of both hip joints based on the angle of the right hip joint and the angle of the left hip joint. The controller 340 may generate a signal for controlling the driver 310 based on the sensed information. For example, the generated signal may be an assistance force for assisting a motion of the user. Alternatively, the generated signal may be a resistance force for impeding a motion of the user. The resistance force may be provided to the user to assist the user in doing a workout. In the following description, a negative magnitude of a workout load (e.g., a torque) may indicate a resistance force, and a positive magnitude thereof may indicate an assistance force.

In an example, the processor 342 of the controller 340 may control the driver 310 to provide the user with a resistance force. For example, the driver 310 may provide the user with a resistance force by applying an active force to the user through the motor 314. Alternatively, the driver 310 may provide the user with a resistance force using the back-drivability of the motor 314, without applying an active force to the user. The back-drivability of the motor may be a responsiveness of the rotation axis of the motor to an external force. When the back-drivability of the motor increases, the motor may more readily respond to an external force acting on the rotation axis of the motor (that is, the rotation axis of the motor may more readily rotate). Even when the same external force is applied to the rotation axis of the motor, the degree of rotation of the rotation axis of the motor may change according to the degree of back-drivability.

According to an example embodiment, the processor 342 of the controller 340 may control the driver 310 to output a torque (or an assisting torque) for assisting a motion of the user. For example, in the hip-type wearable device 300, the driver 310 may be disposed on each of the left hip portion and the right hip portion, and the controller 340 may output a control signal for controlling the driver 310 to generate a torque.

The driver 310 may generate a torque based on the control signal output by the controller 340. A torque value for generating the torque may be externally set or be set by the controller 340. For example, to indicate a magnitude of the torque value, the controller 340 may use a magnitude of a current for the signal transmitted to the driver 310. That is, as the magnitude of the current received by the driver 310 increases, the torque value may increase. As another example, the processor 342 of the controller 340 may transmit the control signal to the motor driver circuit 312 of the driver 310, and the motor driver circuit 312 may generate a current corresponding to the control signal to control the motor 314.

The battery 350 may supply power to the components of the wearable device 300. The wearable device 300 may further include a circuit (e.g., a power management integrated circuit (PMIC)) configured to convert the power of the battery 350 according to an operating voltage of the components of the wearable device 300 and provide the same to the components of the wearable device 300. In addition, the battery 350 may or may not supply power to the motor 314 based on the operation mode of the wearable device 300.

The communication module 352, comprising communication circuitry, may support the establishment of a direct (or wired) communication channel or a wireless communication channel between the wearable device 300 and an external electronic device, and support the communication through the established communication channel. The communication module 352 may include one or more communication processors configured to support direct (or wired) communication or wireless communication. According to an example embodiment, the communication module 352 may include a wireless communication module (e.g., a cellular communication module, a short-range wireless communication module, or a global navigation satellite system (GNSS) communication module) or a wired communication module (e.g., a local area network (LAN) communication module or a power line communication (PLC) module). A corresponding one of these communication modules may communicate with the external electronic device via a first network (e.g., a short-range communication network such as Bluetooth™, wireless-fidelity (Wi-Fi) direct, or infrared data association (IrDA)) or a second network (e.g., a legacy cellular network, a 5G network, a next-generation communication network, the Internet, or a computer network). These various types of communication modules may be implemented as a single component (e.g., a single chip), or may be implemented as multi components (e.g., multi chips) separate from each other.

According to an example embodiment, the electronic device 201 described above with reference to FIG. 2 may be included in the wearable device 300.

According to an example embodiment, the electronic device 201 described above with reference to FIG. 2 may be a separate device physically separated from the wearable device 300, and the electronic device 201 and the wearable device 300 may be connected through short-range wireless communication.

FIG. 4 is a diagram illustrating a wearable device communicating with an electronic device according to an example embodiment.

Referring to FIG. 4, the wearable device 300 (e.g., the wearable device 120 of FIG. 1) described above with reference to FIGS. 3A, 3B, 3C, and 3D may communicate with the electronic device 201 (e.g., the electronic device 110 of FIG. 1) described above with reference to FIG. 2. For example, the electronic device 201 may be an electronic device of a user of the wearable device 300. According to an example embodiment, the wearable device 300 and the electronic device 201 may be connected using a short-range wireless communication method.

The electronic device 201 may display a user interface (UI) for controlling an operation of the wearable device 300 on a display 201-1. The UI may include, for example, at least one soft key through which the user may control the wearable device 300.

The user may input a command for controlling the operation of the wearable device 300 through the UI on the display 201-1 of the electronic device 201, and the electronic device 201 may generate a control instruction corresponding to the command and transmit the generated control instruction to the wearable device 300. The wearable device 300 may operate according to the received control instruction, and transmit a control result to the electronic device 201. The electronic device 201 may display a control completion message on the display 201-1 of the electronic device 201.

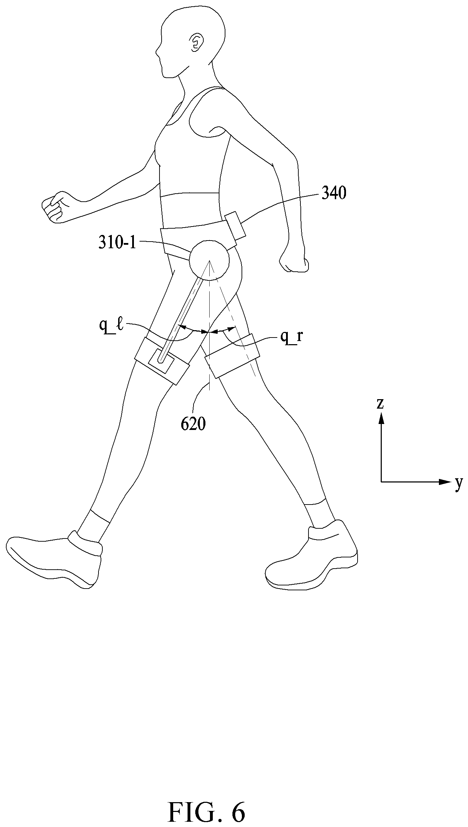

FIGS. 5 and 6 are diagrams illustrating a torque output method of a wearable device according to an example embodiment.

Referring to FIGS. 5 and 6, drivers 310-1 and 310-2 of the wearable device 300 of FIG. 3 (e.g., the wearable device 120 of FIG. 1) may be disposed near the hip joints of a user, and the controller 340 of the wearable device 300 may be disposed near the lower back of the user. The positions of the drivers 310-1 and 310-2 and the controller 340 are not limited to the example positions illustrated in FIGS. 5 and 6.

The wearable device 300 may measure (and/or sense) a left hip joint angle $q\_l$ and a right hip joint angle $q\_r$ of the user. For example, the wearable device 300 may measure the left hip joint angle $q\_l$ of the user through a left encoder and measure the right hip joint angle $q\_r$ of the user through a right encoder. As illustrated in FIG. 6, the left hip angle $q\_l$ may be negative because the left leg of the user is before a reference line 620, and the right hip angle $q\_r$ may be positive because the right leg of the user is behind the reference line 620. According to implementation, the right hip joint angle $q\_r$ may be negative when the right leg is before the reference line 620, and the left hip joint angle $q\_l$ may be positive when the left leg is behind the reference line 620.

According to an example embodiment, the wearable device 300 may obtain a first angle (e.g., $q\_r$) and a second angle (e.g., $q\_l$) by filtering a first raw angle (e.g., $q\_r\_raw$) of a first joint (e.g., the right hip joint) and a second raw angle (e.g., $q\_l\_raw$) of a second joint (e.g., the left hip joint) measured by the sensor unit 320. For example, the wearable device 300 may filter the first raw angle and the second raw angle based on a first previous angle and a second previous angle measured with respect to a previous time.

According to an example embodiment, the wearable device 300 may determine a torque value $\tau(t)$ based on the left hip joint angle $q\_l$, the right hip joint angle $q\_r$, an offset angle $c$, a sensitivity $\alpha$, a gain $\kappa$, and a delay $\Delta t$, and control the motor driver circuit 312 of the wearable device 300 to output the determined torque value $\tau(t)$. The force provided to the user by the torque value $\tau(t)$ may be referred to herein as a force feedback. For example, the wearable device 300 may determine the torque value $\tau(t)$ based on Equation 1 below.

[Equation 1]

$$y = sin(q\_r) - sin(q\_l)$$

$$\tau(t) = \kappa y(t - \Delta t)$$

In Equation 1, $y$ denotes a state factor, $q\_r$ denotes the right hip joint angle, and $q\_l$ denotes the left hip joint angle. According to Equation 1, the state factory may be associated with the distance between the two legs. For example, $y$ being "0" may indicate a state (e.g., a crossing state) in which the distance between the legs is "0", and the absolute value of $y$ being maximum may indicate a state (e.g., a landing state) in which the angle between the legs is maximum or large. According to an example embodiment, when $q\_r$ and $q\_l$ are measured at a time $t$, the state factor may be represented as $y(t)$.

The gain $\kappa$ is a parameter indicating the magnitude and direction of an output torque. As the magnitude of the gain $\kappa$ increases, a greater torque may be output. If the gain $\kappa$ is negative, a torque acting as a resistance force may be output to the user. If the gain $\kappa$ is positive, a torque acting as an assistance force may be output to the user. The delay $\Delta t$ is a parameter associated with a torque output timing. The value of the gain $\kappa$ and the value of the delay $\Delta t$ may be preset, and may be adjustable by a user, the wearable device 300, or the electronic device 201 described above with reference to FIG. 2.

A model for outputting a torque acting as an assistance force to a user using Equation 1 may be defined as a torque output model (e.g., a torque output algorithm). The wearable device 300 or the electronic device 201 may determine the magnitude and delay of a torque to be output by inputting the values of input parameters received through sensors into the torque output model.

According to an example embodiment, the wearable device 300 or the electronic device 201 may determine a first torque value through Equation 2 below by applying a first gain value and a first delay value to a first state factor $y(t)$, wherein the first gain value and the first delay value may be parameter values determined with respect to the state factor $y(t)$.

[Equation 2]

$$\tau_l(t) = \kappa y(t - \Delta t)$$

$$\tau_r(t) = -\kappa y(t - \Delta t)$$

The calculated first torque value may include a value for the first joint and a value for the second joint since it should be applied to the two legs. For example, $\tau_l(t)$ may be a value for the left hip joint, which is the second joint, $\tau_r(t)$ may be a value for the right hip joint, which is the first joint. $\tau_l(t)$ and $\tau_r(t)$ may be values with the same magnitude and opposite torque directions. The wearable device 300 may control the motor driver circuit 312 of the wearable device 300 to output a torque corresponding to the first torque value.

According to an example embodiment, when the user performs an asymmetrical gait with the left leg and the right leg, the wearable device 300 may provide asymmetrical torques respectively to both legs of the user to assist the asymmetric gait (e.g., walk). For example, a stronger assistance force may be provided to a leg with a shorter stride width or a slower swing speed. Hereinafter, a leg with a small stride width or a slow swing speed will be referred to as an affected leg or a target leg.

In general, an affected leg may have a shorter swing time or a smaller stride width than an unaffected leg. According to an example embodiment, a method of adjusting the timing of a torque acting on an affected leg to assist a gait of a user may be considered. For example, an offset angle may be added to an actual joint angle of an affected leg to increase an output time of a torque for assisting a swing motion of the affected leg. $c$ may be the value of a parameter indicating an offset angle between joint angles. As the offset angle is added to the actual joint angle of the affected leg, the value of an input parameter that is input into the torque output model mounted on (and/or applied to) the wearable device 300 may be adjusted. For example, the values of $q\_r$ and $q\_l$ may be adjusted through Equation 3 below. $c_r$ denotes an offset angle with respect to the right hip joint, and $cl$ denotes an offset angle with respect to the left hip joint.

[Equation 3]

$$q_{-r}(t) \leftarrow q_{-r}(t) + c_r$$

$$q_{-l}(t) \leftarrow q_{-l}(t) + c_l$$

According to an example embodiment, the wearable device 300 may filter the state factor to reduce the discomfort the user may experience due to irregular torque outputs. For example, the wearable device 300 or the electronic device 201 may determine an initial state factor $y_{raw}(t)$ of a current time $t$ based on the first angle of the first joint and the second angle of the second joint, and determine the first state factor $y(t)$ based on a previous state factor $y^{prv}$ determined with respect to a previous time $t-1$ and the initial state factor $y_{raw}(t)$. The current time $t$ may be a time at which $t$-th data (e.g., sample) is processed, and the previous time $t-1$ may be a time at which $t-1$-th data is processed. For example, the difference between the current time $t$ and the previous time $t-1$ may be an operation interval of a processor for generating or processing the corresponding items of data. The sensitivity $\alpha$ may be the value of a parameter indicating a sensitivity. For example, the sensitivity value may be continuously adjusted during a test gait. However, the sensitivity value may be preset to a predetermined value to reduce the computational complexity.

According to an example embodiment, a torque output method based on the state factor described with reference to Equation 1 to Equation 3 may be used when the user wearing the wearable device 300 is in a walking state. When the user is in an in-place workout state rather than a walking state, a motion control model corresponding to the workout performed by the user may be used to control the wearable device 300.

FIG. 7 is a flowchart illustrating a method of generating a motion control model for a wearable device according to an example embodiment.

Operations 710 to 760 may be performed by an electronic device (e.g., the electronic device 110 of FIG. 1 or the electronic device 201 of FIG. 2).

In operation 710, the electronic device may load target motion information and basic torque information.

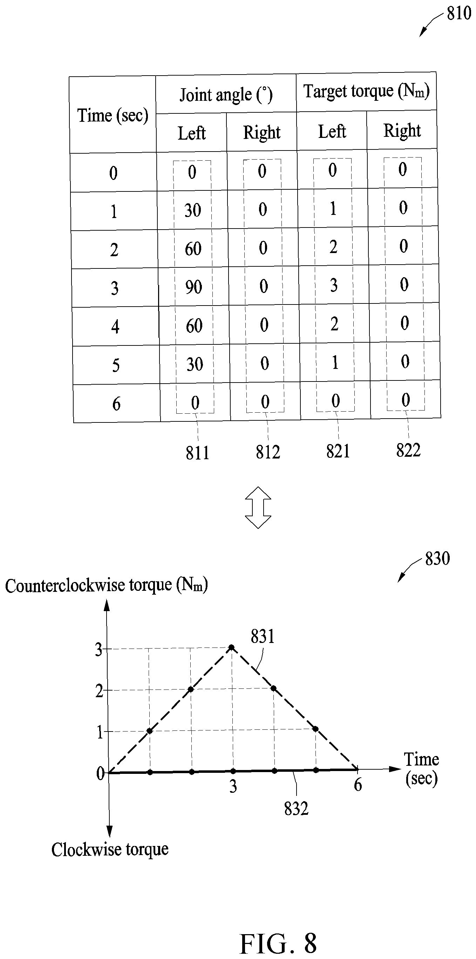

According to an example embodiment, the electronic device may receive target motion information and basic torque information previously generated from a server (e.g., the server 140 of FIG. 1). For example, the electronic device may search for a workout such as a squat, a lunge, or a kickback through the server, and the server may transmit target motion information and basic torque information with respect to the found workout to the electronic device. The target motion information may be a change in a motion of a wearable device (e.g., the wearable device 120 of FIG. 1 and/or the wearable device 300 of FIG. 3) according to a target period. For example, the target motion information may indicate a change in a hip joint angle of the wearable device. The basic torque information may be information about an output torque of the wearable device that is output according to the target period. The target motion information and the basic torque information will be described in detail below with reference to FIG. 8.

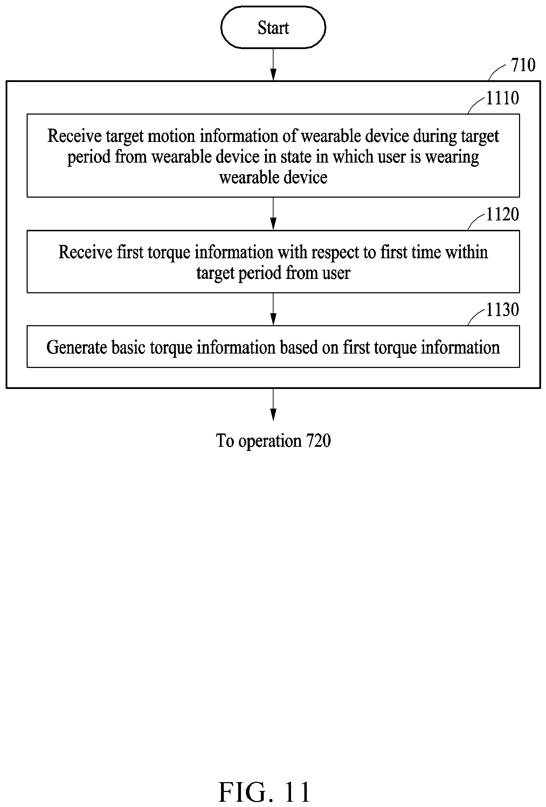

According to an example embodiment, the electronic device may load target motion information and basic torque information generated by a user through the electronic device. A method of generating the target motion information and the basic torque information by the user through the electronic device will be described in detail below with reference to FIGS. 11 to 14.

In operation 720, the electronic device may provide the user with at least one of the target motion information or the basic torque information. For example, the electronic device may simultaneously output the target motion information and the basic torque information through a display. For example, the electronic device may output the basic torque information through the display. The user may identify how a torque is output to the user through the wearable device while performing a workout through the output basic torque information.

According to an example embodiment, the user may control the electronic device such that a torque according to the basic torque information may be output while the user wearing the wearable device is performing a workout corresponding to a target motion. The controlling may be a test on the basic torque information. For example, when a torque output according to the basic torque information is satisfactory, the user may not modify the basic torque information. For example, when a torque output according to the basic torque information is unsatisfactory, the user may modify the basic torque information.

In operation 730, the electronic device may receive an input for modifying at least a portion of the basic torque information from the user.

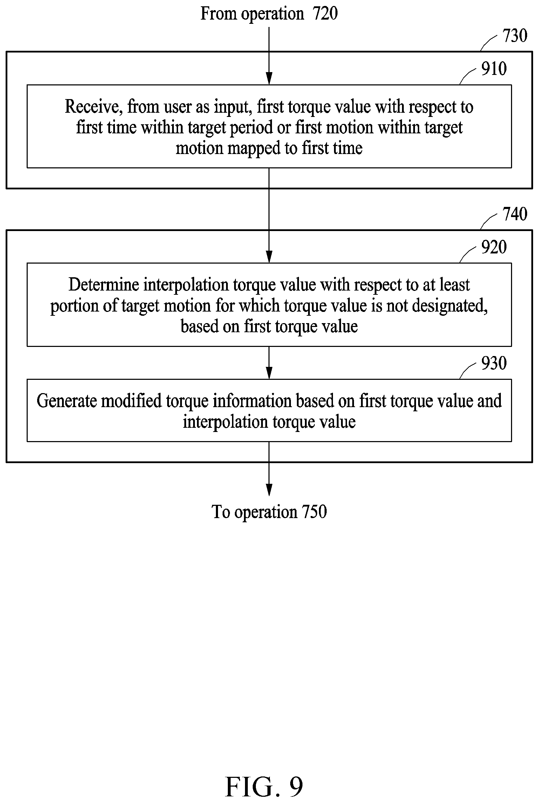

According to an example embodiment, the electronic device may receive, from the user as the input, a first torque value with respect to a first time within the target period or a first motion within the target motion mapped to the first time. A method of receiving the first torque value with respect to the first motion from the user will be described in detail below with reference to FIG. 9.

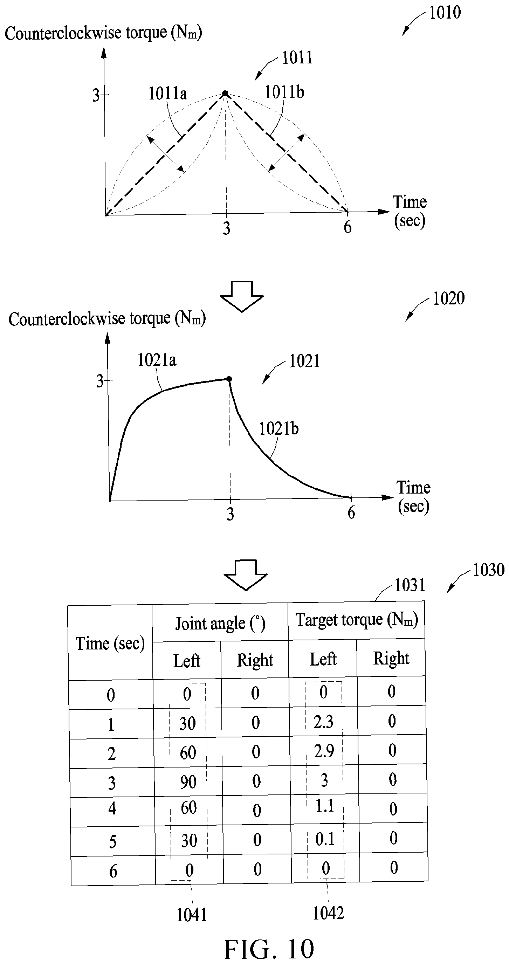

According to an example embodiment, the electronic device may receive, as a modification input with respect to the basic torque information, a drag input of the user with respect to the basic torque information output to the display in the form of a trajectory. A method of receiving the drag input of the user will be described in detail below with reference to FIG. 10.

In operation 740, the electronic device may generate modified torque information based on the basic torque information and the input received from the user.

According to an example embodiment, the electronic device may generate torque information with respect to a motion not received from the user based on torque information with respect to a motion received directly from the user. For example, when a first torque value with respect to a first motion within a target motion is received from the user, the electronic device may determine a second torque value with respect to a second motion based on the first torque value. The electronic device may determine the second torque value so that the torque value to be output may naturally change according to changes in motions. The electronic device may generate the modified torque information based on the first torque value and the second torque value. A method of generating the modified torque information will be described in detail below with reference to FIGS. 9 and 10.

In operation 750, the electronic device may generate a motion control model for controlling the wearable device based on the target motion information and the modified torque information. The generated motion control model may be stored in the electronic device.

According to an example embodiment, the motion control model may be a model that receives motion information (e.g., a joint angle) obtained through a sensor of the wearable device and outputs a value of at least one control parameter for controlling the wearable device to output a desired torque with respect to the corresponding motion.

For example, when a torque output according to the modified torque information is satisfactory, the user may terminate the generation of modified torque information. The modified torque information that has been adjusted may be referred to as final torque information.

For example, when a torque output according to the modified torque information is unsatisfactory, the user may adjust the modified torque information by repeatedly performing operations 730 to 750 described above.

According to an example embodiment, the motion control model may include a first model for a left leg of the wearable device and a second model for a right leg of the wearable device.

In operation 760, the electronic device may provide the user with a workout program by controlling an operation of the wearable device based on the value of the control parameter output by the motion control model.

According to an example embodiment, the electronic device may receive a target workout program received from the user, and determine a target motion control model corresponding to the target workout program from among one or more motion control models stored in the electronic device. The electronic device may receive motion information from the wearable device while the user is performing a workout wearing the wearable device, and control the wearable device to output a torque corresponding to the motion information using the motion information and the motion control model. For example, the torque provided to the user may be output by a motor disposed at a joint (e.g., a hip joint) of the wearable device.

FIG. 8 illustrates target motion information of a wearable device and basic torque information mapped to the target motion information according to an example embodiment.

According to an example embodiment, target motion information of a wearable device (e.g., the wearable device 120 of FIG. 1 or the wearable device 300 of FIG. 3) and basic torque information mapped to the target motion information may be provided to a user in the form of a table 810. The user may visually observe target motion information and basic torque information according to performing a workout. For example, a workout corresponding to the table 810 may be a left knee lift of the user. For example, a target period for one left knee lift may be "6" seconds. The table 810 may include, as target motion information, left joint angle information 811 and right joint angle information 812 during the target period. The table 810 may include, as basic torque information, left target torque information 821 and right target torque information 822 during the target period (or a target motion).

According to an example embodiment, the basic torque information 831 and 832 mapped to the target period (or the target motion information) may be provided to the user in the form of a trajectory (or graph) 830. In the illustrated example, since a joint (e.g., a hip joint or a knee joint) of the right leg basically does not move during the left knee lift, the right joint angle and the value of the target torque may not change.

According to an example embodiment, the target motion information may be provided to the user together with the basic torque information 831 and 832 in the form of a trajectory.

According to an example embodiment, the user may generate a motion control model with respect to the target motion information and the basic torque information through an electronic device (e.g., the electronic device 110 of FIG. 1 or the electronic device 201 of FIG. 2), and perform a workout using the generated motion control model wearing the wearable device. For example, when a torque output of the wearable device according to the motion control model is unsatisfactory, the user may modify the basic torque information.

For example, the target motion information and the basic torque information may be information generated directly by the user of the wearable device. For example, the target motion information and the basic torque information may be information generated by another user other than the user of the wearable device. For example, the target motion information and the basic torque information may be information received by the electronic device from a server (e.g., the server 140 of FIG. 1).

FIG. 9 is a flowchart illustrating a method of generating modified torque information based on an input received from a user according to an example embodiment.

According to an example embodiment, operation 730 described above with reference to FIG. 7 may include operation 910 to be described hereinafter.

In operation 910, an electronic device (e.g., the electronic device 110 of FIG. 1 or the electronic device 201 of FIG. 2) may receive, from a user as an input, a first torque value with respect to a first time within a target period or a first motion within a target motion mapped to the first time.

For example, in the table 810 described above with reference to FIG. 8, a basic torque value with respect to a first time (e.g., "2" seconds) or a first motion (e.g., the left joint angle of 60 degrees) within a target motion mapped to the first time is "2" Nm, and the user may input "2.9" Nm as a first torque value. The user may input the first torque value in a manner of increasing or decreasing the value from the basic torque value.

According to an example embodiment, operation 740 described above with reference to FIG. 7 may include operations 920 and 930 to be described hereinafter. Operation 920 may be performed after operation 910 is performed.

In operation 920, the electronic device may determine an interpolation torque value with respect to at least a portion of the target motion for which a torque value is not designated, based on the first torque value.

For example, when a torque value with respect to a target motion (e.g., the left joint angle of 30 degrees) corresponding to "1" second in the left joint angle information 811 of the table 810 described with reference to FIG. 8 is not designated by the user, the electronic device may determine an interpolation torque value with respect to the target motion. For example, when "2.9" Nm is set with respect to "2" seconds and "3" Nm is set with respect to "3" seconds, the electronic device may determine, to be an interpolation torque value (e.g., "2.3" Nm), the torque value with respect to the target motion corresponding to "1" second based on an aspect of a change in the target motion and set torque values.

In operation 930, the electronic device may generate modified torque information based on the first torque value and the interpolation torque value. For example, the modified torque information may be torque information output by a wearable device (e.g., the wearable device 120 of FIG. 1 or the wearable device 300 of FIG. 3) during the target motion (or during the target period).

FIG. 10 illustrates modified torque information generated based on basic torque information and an input of a user according to an example embodiment.

In operation 1010, an electronic device (e.g., the electronic device 110 of FIG. 1 or the electronic device 201 of FIG. 2) may provide a user with basic torque information in the form of a trajectory 1011.

For example, the user may set, to "3" Nm, a torque value output at "3" seconds at which an aspect of a motion changes. A point at which the aspect of the motion changes may be referred to as a feature point. It may be divided based on "3" seconds into a first period in which the torque value increases and a second period in which the torque value decreases. Although it is divided based on "3" seconds into the first period and the second period in the illustrated embodiment, the number of divided periods may vary depending on an embodiment.

In operation 1020, the user may input, into the electronic device, a drag input of modifying a trajectory 1011a of first basic torque information corresponding to the first period or a trajectory 1011b of second basic torque information corresponding to the second period.

According to an example embodiment, a trajectory 1021 of modified torque information modified by the user is shown. For example, the user may move a trajectory 1021a of first modified torque information by dragging the trajectory 1011a of the first basic torque information upward to increase the torque in the first period. For example, the user may move a trajectory 1021b of second modified torque information by dragging the trajectory 1011b of the second basic torque information downward to decrease the torque in the second period. The electronic device may change the shape of the trajectory based on the drag input of the user. The shape of the trajectory may be determined so that the torque value continuously changes within the corresponding period.

According to the trajectory 1021 of the modified torque information, a counterclockwise strong torque may be provided to the user as a workout load from the start portion in the first period in which the user lifts the left knee, and the counterclockwise torque may be quickly decreased from the start portion in the second period in which the user lowers the left knee, whereby the torque may be prevented from acting as an assistance force for lowering the left knee.

In operation 1030, the electronic device may convert the trajectory 1021 of the modified torque information into the form of a table 1031. The table 1031 may include, as target motion information, left joint angle information 1041 during the target period and left target motion information 1042 during the target period (or during the target motion). For example, the left target torque information 1042 may correspond to the trajectory 1021 of the modified torque information.

According to an example embodiment, the electronic device may generate a motion control model based on the table 1031.

FIG. 11 is a flowchart illustrating a method of loading target motion information and basic torque information according to an example embodiment.

According to an example embodiment, operation 710 described above with reference to FIG. 7 may include operations 1110 to 1130 to be described hereinafter. For example, operations 1110 to 1130 may relate to a method of generating basic torque information by a user of a wearable device (e.g., the wearable device 120 of FIG. 1 or the wearable device 300 of FIG. 3). Operations 710 to 760 may be performed by an electronic device (e.g., the electronic device 110 of FIG. 1 or the electronic device 201 of FIG. 2).

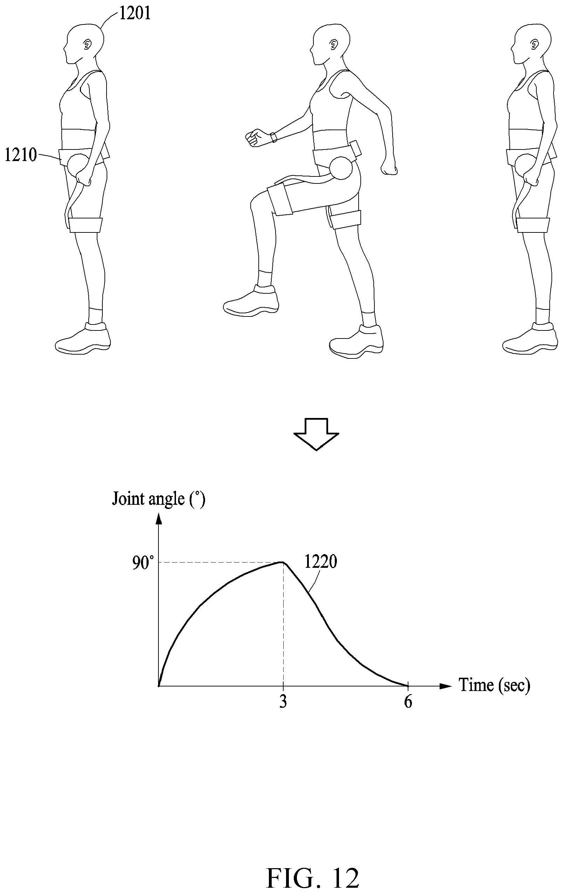

In operation 1110, the electronic device may receive target motion information of the wearable device during a target period from the wearable device in a state in which the user is wearing the wearable device. A method of receiving the target motion information from the wearable device by the electronic device will be described in detail below with reference to FIG. 12.

According to an example embodiment, in a state in which the electronic device and the wearable device are connected (or paired) through wireless communication, the user wearing the wearable device may perform a desired workout during a target period. The wearable device may generate information on a motion performed for the target period through a sensor, and transmit the generated motion information to the electronic device. For example, the motion information may be the angle of a hop j oint of the user. A method of receiving the target motion information by the electronic device will be described in detail below with reference to FIG. 12.

In operation 1120, the electronic device may receive first torque information with respect to the first time within the target period from the user.

According to an example embodiment, while the target motion information is generated by the wearable device the user is wearing, the electronic device may receive first torque information from the user and associate a first motion corresponding to a time (e.g., the first time) at which the first torque information is received with the first torque information. For example, the first torque information may include a torque output direction (a clockwise or counterclockwise direction). For example, the first torque information may include a magnitude of an output torque.

According to an example embodiment, the electronic device may further include a microphone for receiving an utterance of the user. The electronic device may generate the first torque information based on the received utterance.

According to an example embodiment, after the target motion information is generated, the user may input first torque information on a desired first motion (or first time) into the electronic device while observing the target motion information.

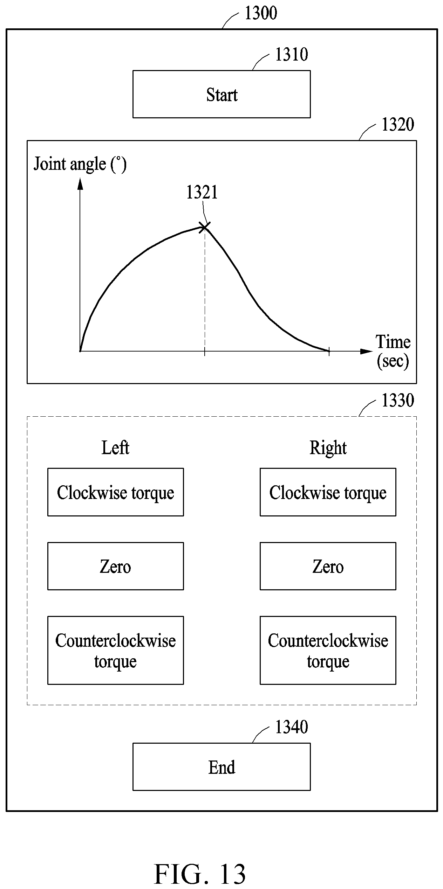

A method of receiving the first torque information with respect to the first time within the target period will be described in detail below with reference to FIG. 13.

In operation 1130, the electronic device may generate basic torque information (e.g., the target torque information 821 of FIG. 8) based on the first torque information.

FIG. 12 is a flowchart illustrating a method of receiving target motion information using a wearable device according to an example embodiment.

According to an example embodiment, a user 1201 may perform a target motion wearing a wearable device 1210 (e.g., the wearable device 120 of FIG. 1 or the wearable device 300 of FIG. 3). For example, the user may perform a left knee lift as the target motion. For example, the target motion may be performed for "6" seconds.

While the user is performing the target motion, the wearable device 1210 may generate motion information 1220 using a sensor. For example, the motion information 1220 may be a trajectory of the angle of the left hip joint of the user. The wearable device 1210 may transmit the generated motion information 1220 to an electronic device (e.g., the electronic device 110 of FIG. 1 or the electronic device 201 of FIG. 2).

FIG. 13 illustrates a user interface (UI) screen for receiving torque information with respect to target motion information from a user according to an example embodiment.

According to an example embodiment, a user wearing a wearable device (e.g., the wearable device 120 of FIG. 1, the wearable device 300 of FIG. 3, or the wearable device 1210 of FIG. 12) may perform a target motion by touching a start button 1310 on a UI screen 1300 that is output by an electronic device (e.g., the electronic device 110 of FIG. 1 or the electronic device 201 of FIG. 2).

The electronic device may output, on the UI screen 1300, motion information 1320 received from the wearable device while the user is performing the target motion.

While performing the target motion, the user may input torque information into the electronic device with respect to a motion (or a time) (e.g., a first motion 1321) for which a torque output is desired. A point corresponding to the motion for which a torque output is desired may be a feature point. For example, first torque information corresponding to the first motion 1321 may be input through a torque information input interface 1330 provided on the UI screen 1300. For example, the first torque information may include a direction of an output torque. For example, the first torque information may include a magnitude of an output torque.

According to an example embodiment, the user may input the first torque information into the electronic device not only while performing the target motion, but also after performing the target motion.

The user may terminate the generation of the target motion information and the basic torque information by touching an end button 1340 on the UI screen 1300.

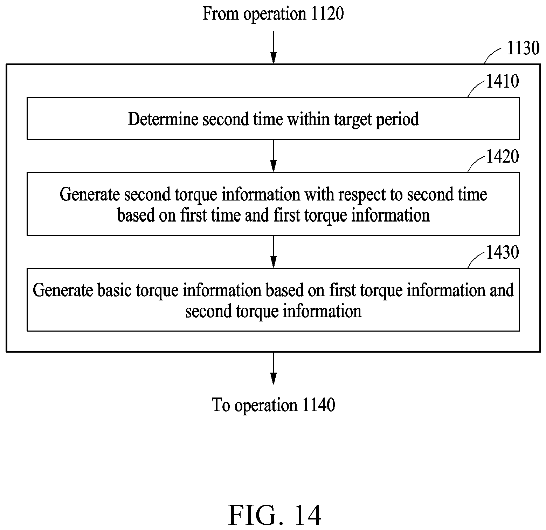

FIG. 14 is a flowchart illustrating a method of generating basic torque information based on first torque information and second torque information according to an example embodiment.

According to an example embodiment, operation 1130 described above with reference to FIG. 11 may include operations 1410 to 1430 to be described hereinafter. Operations 1410 to 1430 may be performed by an electronic device (e.g., the electronic device 110 of FIG. 1 or the electronic device 201 of FIG. 2).

In operation 1410, the electronic device may determine a second time within the target period. For example, the second time may be a time not designated by the user.

In operation 1420, the electronic device may generate second torque information with respect to the second time based on the first time and the first torque information. For example, when the second time is between first times designated by the user, the second torque information may be generated based on pieces of torque information corresponding to the first times. For example, the second torque information may be generated as a trajectory connecting the pieces of torque information corresponding to the first times.

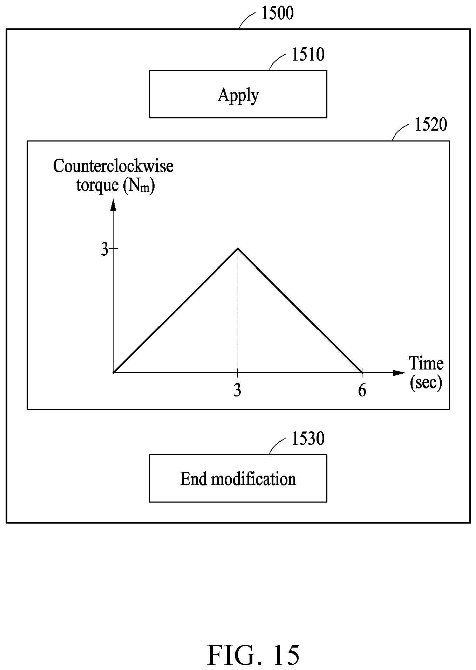

FIG. 15 illustrates a UI screen for generating modified torque information based on basic torque information according to an example embodiment.

According to an example embodiment, a UI screen 1500 output by an electronic device (e.g., the electronic device 110 of FIG. 1 or the electronic device 201 of FIG. 2) for generating modified torque information based on basic torque information may be provided to a user in operation 720 described above with reference to FIG. 7.

The user may identify modified torque information (or basic torque information) 1520 output on the UI screen 1500. When the modified torque information 1520 is modified, modified torque information may be output on the UI screen 1500.

According to an example embodiment, the user may test the modified torque information 1520 wearing a wearable device (e.g., the wearable device 120 of FIG. 1, the wearable device 300 of FIG. 3, or the wearable device 1210 of FIG. 12). For example, the user may touch an application button 1510 to test the modified torque information 1520 output on the UI screen 1500. When the application button 1510 is touched, the electronic device may generate a motion control model corresponding to the modified torque information 1520 and control an operation of the wearable device based on the motion control model.

When a torque output by the wearable device is unsatisfactory, the user may repeatedly modify the modified torque information 1520. The user may touch an end-modification button 1530 when the torque output by the wearable device is satisfactory. When the end-modification button 1530 is touched, the electronic device may generate a motion control model corresponding to the modified torque information 1520 and store the motion control model. The generated motion control model corresponding to the modified torque information 1520 may be a motion control model personalized to the user.

FIG. 16 is a diagram illustrating a configuration of a server according to an example embodiment.

A server 1600 may include a communicator 1610 comprising communication circuitry, a processor 1620 (comprising processing circuitry), and a memory 1630. For example, the server 1600 may be the server 140 described above with reference to FIG. 1.

The communicator 1610, comprising communication circuitry, may be connected, directly or indirectly, to the processor 1620 and the memory 1630 and transmit and receive data to and from the processor 1620 and the memory 1630. The communicator 1610 may be connected, directly or indirectly, to another external device and transmit and receive data to and from the external device.

The communicator 1610 may be implemented as a circuitry in the server 1600. For example, the communicator 1610 may include an internal bus and an external bus. In another example, the communicator 1610 may be an element that connects the server 1600 and the external device. The communicator 1610 may be an interface. The communicator 1610 may receive data from the external device and transmit the data to the processor 1620 and the memory 1630.

The processor 1620, comprising processing circuitry, may process the data received by the communicator 1610 and data stored in the memory 1630. A processor described herein may be a hardware-implemented processing device having a physically structured circuit to execute desired operations. The desired operations may include, for example, code or instructions included in a program. The hardware-implemented data processing device may include, for example, a microprocessor, a central processing unit (CPU), a processor core, a multi-core processor, a multiprocessor, an application-specific integrated circuit (ASIC), and a field-programmable gate array (FPGA). Each processor herein comprises processing circuitry.

The processor 1620 may execute computer-readable code (e.g., software) stored in a memory (e.g.,

the memory 1630) and instructions triggered by the processor 1620.

The memory 1630 may store therein the data received by the communicator 1610 and the data processed by the processor 1620. For example, the memory 1630 may store the program (or an application, or software). The stored program may be a set of syntax coded and executable by the processor 1620 to transmit basic torque information corresponding to a workout program selected by the user from among a plurality of workout programs to the electronic device.

According to an example embodiment, the memory 1630 may include at least one volatile memory, nonvolatile memory, random-access memory (RAM), flash memory, a hard disk drive, and an optical disc drive.

The memory 1630 may store an instruction set (e.g., software) for operating the server 1600. The instruction set for operating the server 1600 may be executed by the processor 1620. According to an example embodiment, the memory 1630 may include a database including basic torque information for each of the plurality of workout programs.

According to an example embodiment, an electronic device may include a communication module configured to exchange data with an external device, and at least one processor configured to control the electronic device, wherein the processor may be configured to provide a user of the electronic device with at least one of target motion information of a wearable device according to a target period or basic torque information mapped to the target motion information and output by the wearable device, receive an input for modifying at least a portion of the basic torque information from the user, generate modified torque information based on the basic torque information and the input, and generate a motion control model for controlling the wearable device based on the target motion information and the modified torque information, wherein an operation of the wearable device may be controlled based on the motion control model.

According to an example embodiment, the electronic device may further include a display configured to output the target motion information and the basic torque information to the user.

According to an example embodiment, the processor may be configured to receive, as the input, a drag input of the user with respect to the basic torque information output to the display in the form of a trajectory.

Each embodiment herein may be used in combination with any other embodiment(s) described herein.

According to an example embodiment, the processor may be configured to receive, from the user as the input, a first torque value with respect to a first time within the target period or a first motion within the target motion mapped to the first time.

According to an example embodiment, the processor may be configured to determine an interpolation torque value with respect to at least a portion of the target motion for which a torque value is not designated, based on the first torque value, and generate the modified torque information based on the first torque value and the interpolation torque value. "Based on" as used herein covers based at least on.

According to an example embodiment, the processor may be configured to generate the motion control model so that, when the user performs the target motion while wearing the wearable device, the wearable device may output a torque based on the modified torque information during the target motion.

According to an example embodiment, the torque may be output by a motor disposed in a joint of the wearable device.

According to an example embodiment, the motion control model may include a first model for a left leg of the wearable device and a second model for a right leg of the wearable device.

According to an example embodiment, the processor may be configured to receive the target motion information of the wearable device during the target period from the wearable device in a state in which the user is wearing the wearable device, receive first torque information with respect to the first time within the target period from the user, and generate the basic torque information based on the first torque information.

According to an example embodiment, the processor may be configured to determine a second time within the target period, generate second torque information with respect to the second time based on the first time and the first torque information, and generate the basic torque information based on the first torque information and the second torque information.

According to an example embodiment, the first torque information may include at least one of direction information or magnitude information of a torque output at the first time.

According to an example embodiment, the electronic device may further include a microphone configured to receive an utterance of the user, wherein the processor is configured to generate the first torque information based on the utterance.

According to an example embodiment, a method of generating a motion control model for controlling a wearable device, performed by an electronic device, may include providing a user of the electronic device with at least one of target motion information of the wearable device according to a target period or basic torque information mapped to the target motion information and output by the wearable device, receiving an input for modifying at least a portion of the basic torque information from the user, generating modified torque information based on the basic torque information and the input, and generating a motion control model for controlling the wearable device based on the target motion information and the modified torque information, wherein an operation of the wearable device may be controlled based on the motion control model.

According to an example embodiment, the receiving of the input for modifying at least a portion of the basic torque information from the user may include receiving, as the input, a drag input of the user with respect to the basic torque information output to the display in the form of a trajectory.

According to an example embodiment, the receiving of the input for modifying at least a portion of the basic torque information from the user may include receiving, from the user as the input, a first torque value with respect to a first time within the target period or a first motion within the target motion mapped to the first time.

According to an example embodiment, the generating of the modified torque information based on the basic torque information and the input may include determining an interpolation torque value with respect to at least a portion of the target motion for which a torque value is not designated, based on the first torque value, and generating the modified torque information based on the first torque value and the interpolation torque value.

According to an example embodiment, the generating of the motion control model may include generating the motion control model so that, when the user performs the target motion while wearing the wearable device, the wearable device outputs a torque based on the modified torque information during the target motion.

According to an example embodiment, the method may further include receiving the target motion information of the wearable device during the target period from the wearable device in a state in which the user is wearing the wearable device, receiving first torque information with respect to the first time within the target period from the user, and generating the basic torque information based on the first torque information.

According to an example embodiment, the generating of the basic torque information based on the first torque information may include determining a second time within the target period, and generating second torque information with respect to the second time based on the first time and the first torque information, wherein the basic torque information may include the first torque information and the second torque information.

According to an example embodiment, an electronic device may include a communication module configured to exchange data with an external device, and at least one processor configured to control the electronic device, wherein the processor may be configured to receive target motion information of the wearable device during a target period from the wearable device in a state in which a user is wearing the wearable device, receive first torque information with respect to a first time within the target period from the user, generate basic torque information based on the first torque information, and generate a motion control model for controlling the wearable device based on the target motion information and the basic torque information, wherein an operation of the wearable device may be controlled based on the motion control model.

The embodiments described herein may be implemented using a hardware component, a software component and/or a combination thereof. A processing device may be implemented using one or more general-purpose or special-purpose computers, such as, for example, a processor, a controller and an arithmetic logic unit (ALU), a DSP, a microcomputer, a field-programmable gate array (FPGA), a programmable logic unit (PLU), a microprocessor or any other device capable of responding to and executing instructions in a defined manner. The processing device may run an operating system (OS) and one or more software applications that run on the OS. The processing device also may access, store, manipulate, process, and create data in response to execution of the software. For purpose of simplicity, the description of a processing device is used as singular; however, one skilled in the art will appreciate that a processing device may include multiple processing elements and multiple types of processing elements. For example, the processing device may include a plurality of processors, or a single processor and a single controller. In addition, different processing configurations are possible, such as parallel processors.

The software may include a computer program, a piece of code, an instruction, or some combination thereof, to independently or uniformly instruct or configure the processing device to operate as desired. Software and data may be embodied permanently or temporarily in any type of machine, component, physical or virtual equipment, computer storage medium or device, or in a propagated signal wave capable of providing instructions or data to or being interpreted by the processing device. The software also may be distributed over network-coupled computer systems so that the software is stored and executed in a distributed fashion. The software and data may be stored by one or more non-transitory computer-readable recording mediums.

The methods according to the above-described embodiments may be recorded in non-transitory computer-readable media including program instructions to implement various operations of the above-described embodiments. The media may also include, alone or in combination with the program instructions, data files, data structures, and the like. The program instructions recorded on the media may be those specially designed and constructed for the purposes of embodiments, or they may be of the kind well-known and available to those having skill in the computer software arts. Examples of non-transitory computer-readable media include magnetic media such as hard disks, floppy disks, and magnetic tape; optical media such as CD-ROM discs and/or DVDs; magneto-optical media such as optical discs; and hardware devices that are specially configured to store and perform program instructions, such as read-only memory (ROM), random access memory (RAM), flash memory, and the like. Examples of program instructions include both machine code, such as produced by a compiler, and files containing higher-level code that may be executed by the computer using an interpreter.

The above-described devices may be configured to act as one or more software modules in order to perform the operations of the above-described examples, or vice versa. The term "software module" as used herein may include various processing circuitry and/or executable program instructions. The same applies to "software modules."

As described above, although the embodiments have been described with reference to the limited drawings, a person skilled in the art may apply various technical modifications and variations based thereon. For example, suitable results may be achieved if the described techniques are performed in a different order and/or if components in a described system, architecture, device, or circuit are combined in a different manner and/or replaced or supplemented by other components or their equivalents.

While the disclosure has been illustrated and described with reference to various embodiments, it will be understood that the various embodiments are intended to be illustrative, not limiting. It will further be understood by those skilled in the art that various changes in form and detail may be made without departing from the true spirit and full scope of the disclosure, including the appended claims and their equivalents. It will also be understood that any of the embodiment(s) described herein may be used in conjunction with any other embodiment(s) described herein.

## Claims

1. An electronic device, comprising:

    a communication module, comprising communication circuitry, configured to exchange data with an external device; and
    at least one processor configured to control the electronic device,
    wherein the at least one processor is configured to:

        provide a user of the electronic device with at least one of target motion information of a wearable device based on a target period or basic torque information mapped to at least the target motion information,
        receive an input for modifying at least a portion of the basic torque information from the user,
        generate modified torque information based on the basic torque information and the input, and
        generate a motion control model for controlling the wearable device based on the target motion information and the modified torque information, wherein an operation of the wearable device is to be controlled based on the motion control model.

2. The electronic device of claim 1, further comprising:
a display configured to output the target motion information and the basic torque information to the user.

3. The electronic device of claim 2, wherein
the at least one processor is configured to receive, as at least part of the input, a drag input of the user with respect to the basic torque information output to the display in the form of a trajectory.

4. The electronic device of claim 2, wherein
the at least one processor is configured to receive, from the user as at least part of the input, a first torque value with respect to a first time within the target period and/or a first motion within the target motion mapped to the first time.

5. The electronic device of claim 4, wherein
the at least one processor is configured to:

    determine an interpolation torque value with respect to at least a portion of the target motion for which a torque value is not designated, based on the first torque value, and
    generate the modified torque information based on the first torque value and the interpolation torque value.

6. The electronic device of claim 1, wherein
the at least one processor is configured to generate the motion control model so that, when the user performs the target motion while wearing the wearable device, the wearable device is to output a torque based on the modified torque information during the target motion.

7. The electronic device of claim 1, wherein
the torque is to be output and/or generated by at least a motor disposed in a joint of the wearable device.

8. The electronic device of claim 1, wherein
the motion control model comprises a first model for a left leg of the wearable device and a second model for a right leg of the wearable device.

9. The electronic device of claim 1, wherein
the at least one processor is configured to:

    receive the target motion information of the wearable device during the target period from the wearable device in a state in which the user is wearing the wearable device,
    receive first torque information with respect to the first time within the target period from the user, and
generate the basic torque information based on the first torque information.

10. The electronic device of claim 9, wherein the at least one processor is configured to:

determine a second time within the target period, generate second torque information with respect to the second time based on the first time and the first torque information, and generate the basic torque information based on the first torque information and the second torque information.

11. The electronic device of claim 9, wherein the first torque information comprises at least one of direction information or magnitude information of a torque output at the first time.

12. The electronic device of claim 9, further comprising:

a microphone configured to receive an utterance of the user,
wherein the at least one processor is configured to generate the first torque information based on the utterance.

13. A method of generating a motion control model for controlling a wearable device, performed by an electronic device, the method comprising:

providing a user of the electronic device with at least one of target motion information of the wearable device based on a target period, or basic torque information mapped to the target motion information and output by the wearable device;
receiving an input for modifying at least a portion of the basic torque information from the user;
generating modified torque information based on the basic torque information and the input; and
generating a motion control model for controlling the wearable device based on the target motion information and the modified torque information, wherein an operation of the wearable device is to be controlled based on the motion control model.

14. The method of claim 13, wherein the receiving of the input for modifying at least a portion of the basic torque information from the user comprises receiving, as at least part of the input, a drag input of the user with respect to the basic torque information output to the display in the form of a trajectory.

15. The method of claim 13, wherein the receiving of the input for modifying at least a portion of the basic torque information from the user comprises receiving, from the user as at least part of the input, a first torque value with respect to a first time within the target period and/or a first motion within the target motion mapped to the first time.

FIG. 1

FIG. 2

EP 4 360 813 A1

FIG. 3A

FIG. 3B

300

```
┌──────────┐  ┌──────────────┐  ┌──────────────┐  ┌──────────┐
│ Memory   │  │ Communication│  │Input interface│  │ Battery  │
│  344     │  │ module 352   │  │   346        │  │  350     │
└──────────┘  └──────────────┘  └──────────────┘  └──────────┘
```

┌──────────┐  ┌────────────────────────────────────┐  ┌────────────────────┐
│ Sensor   │  │            Processor               │  │ Motor driver circuit│
│  321     │  │             342                    │  │        312          │
└──────────┘  └────────────────────────────────────┘  └────────────────────┘

┌────────────────────┐
│      Motor         │
│       314          │
└────────────────────┘

FIG. 3C

300-1

```
                        ┌──────────────┐
                        │   Battery    │
                        │     350      │
                        └──────┬───────┘
         ┌──────────────┬──────┼──────────┬──────────────┐
         │              ▼      │          ▼              │
         │      ┌──────────────┐│  ┌──────────────┐      │
         │      │   Memory     ││  │Input interface│     │
         │      │    344       ││  │    346       │      │
         │      └──────┬───────┘│  └──────┬───────┘      │
         │             │        ▼         │              │
         │             │ ┌──────────────────┐            │
  ┌──────▼──────┐      │ │                  │    ┌───────▼───────┐
  │Motor driver │──────┴─│   Processor      │────│ Motor driver  │
  │circuit 312  │        │     342          │    │ circuit 312-1 │
  └──────┬──────┘        └──────────────────┘    └───────┬───────┘
         │                                               │
  ┌──────▼──────┐                                 ┌──────▼──────┐
  │   Motor     │                                 │   Motor     │
  │    314      │                                 │   314-1     │
  └─────────────┘                                 └─────────────┘
  ┌─────────────┐                                 ┌─────────────┐
  │  Sensor     │                                 │  Sensor     │
  │    321      │                                 │   321-1     │
  └─────────────┘                                 └─────────────┘
```

FIG. 3D

201-1

Electronic device 201

............
............
............
............
............
............

Wearable device
300

Control instruction

Electronic device 201

201-1

Control result

Wearable device
300

Control completed

FIG. 4

FIG. 5

FIG. 6

Start

710

Load target motion information and basic torque information

720

Provide user with target motion information and basic torque information

730

Receive, from user, input for modifying at least portion of
basic torque information

740

Generate modified torque information based on basic torque information and input

750

Generate motion control model for controlling wearable device based on
target motion information and modified torque information

760

Control operation of wearable device based on motion control model

End

FIG. 7

810

| Time (sec) | Joint angle (°) | | Target torque (Nm) | |
|---|---|---|---|---|
| | Left | Right | Left | Right |
| 0 | 0 | 0 | 0 | 0 |
| 1 | 30 | 0 | 1 | 0 |
| 2 | 60 | 0 | 2 | 0 |
| 3 | 90 | 0 | 3 | 0 |
| 4 | 60 | 0 | 2 | 0 |
| 5 | 30 | 0 | 1 | 0 |
| 6 | 0 | 0 | 0 | 0 |

811      812      821      822

⇕

830

FIG. 8

From operation 720

```
                                                          /‒ 730
┌──────────────────────────────────────────────────────────┐
│                                                  /‒ 910   │
│  ┌───────────────────────────────────────────────────┐   │
│  │  Receive, from user as input, first torque value   │   │
│  │  with respect to first time within target period   │   │
│  │  or first motion within target motion mapped to    │   │
│  │                    first time                      │   │
│  └───────────────────────────────────────────────────┘   │
└──────────────────────────────────────────────────────────┘

                                                          /‒ 740
┌──────────────────────────────────────────────────────────┐
│                                                  /‒ 920   │
│  ┌───────────────────────────────────────────────────┐   │
│  │  Determine interpolation torque value with respect │   │
│  │  to at least portion of target motion for which    │   │
│  │  torque value is not designated, based on first    │   │
│  │                  torque value                      │   │
│  └───────────────────────────────────────────────────┘   │
│                                                  /‒ 930   │
│  ┌───────────────────────────────────────────────────┐   │
│  │  Generate modified torque information based on     │   │
│  │  first torque value and interpolation torque value │   │
│  └───────────────────────────────────────────────────┘   │
└──────────────────────────────────────────────────────────┘
```

To operation 750

# FIG. 9

1010

Counterclockwise torque (Nm)

1011

1011a    1011b

3

Time (sec)

3    6

⇩

1020

Counterclockwise torque (Nm)

1021a    1021

3

1021b

Time (sec)

3    6

⇩

1031    1030

| Time (sec) | Joint angle (°) | | Target torque (Nm) | |
|---|---|---|---|---|
| | Left | Right | Left | Right |
| 0 | 0 | 0 | 0 | 0 |
| 1 | 30 | 0 | 2.3 | 0 |
| 2 | 60 | 0 | 2.9 | 0 |
| 3 | 90 | 0 | 3 | 0 |
| 4 | 60 | 0 | 1.1 | 0 |
| 5 | 30 | 0 | 0.1 | 0 |
| 6 | 0 | 0 | 0 | 0 |

1041    1042

FIG. 10

Start

710

1110

Receive target motion information of wearable device during target period from wearable device in state in which user is wearing wearable device

1120

Receive first torque information with respect to first time within target period from user

1130

Generate basic torque information based on first torque information

To operation 720

FIG. 11

FIG. 12

FIG. 13

From operation 1120

1130

1410

Determine second time within target period

1420

Generate second torque information with respect to second time based on first time and first torque information

1430

Generate basic torque information based on first torque information and second torque information

To operation 1140

FIG. 14

1500

1510

Apply

1520

Counterclockwise
torque (Nm)

3

3    6    Time
(sec)

1530

End modification

FIG. 15

FIG. 16

## INTERNATIONAL SEARCH REPORT

| International application No. |
| --- |
| **PCT/KR2022/019609** |

| A. | CLASSIFICATION OF SUBJECT MATTER |
| --- | --- |

B25J 9/00(2006.01)i; B25J 9/16(2006.01)i; B25J 13/00(2006.01)i; B25J 13/06(2006.01)i; B25J 13/08(2006.01)i

According to International Patent Classification (IPC) or to both national classification and IPC

| B. | FIELDS SEARCHED |
| --- | --- |

Minimum documentation searched (classification system followed by classification symbols)

B25J 9/00(2006.01); A61B 5/11(2006.01); A61H 3/00(2006.01); A63B 21/00(2006.01); A63B 21/005(2006.01); B25J 11/00(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 웨어러블(wearable), 운동(workout), 매핑(mapping), 토크 정보(torque information), 수정(modification), 움직임 제어 모델(motion control model), 드래그(drag)

| C. | DOCUMENTS CONSIDERED TO BE RELEVANT |
| --- | --- |

| Category* | Citation of document, with indication, where appropriate, of the relevant passages | Relevant to claim No. |
| --- | --- | --- |
| A | KR 10-2021-0054086 A (SAMSUNG ELECTRONICS CO., LTD.) 13 May 2021 (2021-05-13)<br>See paragraphs [0030]-[0053] and [0058] and figures 1-4. | 1-15 |
| A | KR 10-2021-0050636 A (SAMSUNG ELECTRONICS CO., LTD.) 10 May 2021 (2021-05-10)<br>See paragraphs [0039], [0042], [0043], [0047], [0049], [0052] and [0055]-[0059] and figures 3 and 6. | 1-15 |
| A | KR 10-2017-0083829 A (HANWHA TECHWIN CO., LTD.) 19 July 2017 (2017-07-19)<br>See paragraphs [0055], [0056], [0074], [0078] and [0094] and figures 4 and 6. | 1-15 |
| A | US 11148279 B1 (DEPHY, INC.) 19 October 2021 (2021-10-19)<br>See column 16, line 44 – column 37, line 59 and figures 14-16. | 1-15 |
| A | KR 10-2018-0136656 A (SAMSUNG ELECTRONICS CO., LTD.) 26 December 2018 (2018-12-26)<br>See paragraphs [0016], [0041], [0042], [0054]-[0058] and [0065] and figures 1-4. | 1-15 |

☐ Further documents are listed in the continuation of Box C.  ☑ See patent family annex.

| * | Special categories of cited documents: |
| --- | --- |
| "A" | document defining the general state of the art which is not considered to be of particular relevance |
| "D" | document cited by the applicant in the international application |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
| --- | --- |
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
| --- | --- |
| **10 March 2023** | **10 March 2023** |

| Name and mailing address of the ISA/KR | Authorized officer |
| --- | --- |
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

**INTERNATIONAL SEARCH REPORT**
Information on patent family members

International application No.

**PCT/KR2022/019609**

| Patent document cited in search report | | | Publication date (day/month/year) | Patent family member(s) | | | Publication date (day/month/year) |
|---|---|---|---|---|---|---|---|
| KR | 10-2021-0054086 | A | 13 May 2021 | CN | 112774115 | A | 11 May 2021 |
| | | | | EP | 3815666 | A1 | 05 May 2021 |
| | | | | US | 2021-0128972 | A1 | 06 May 2021 |
| KR | 10-2021-0050636 | A | 10 May 2021 | US | 2021-0121729 | A1 | 29 April 2021 |
| KR | 10-2017-0083829 | A | 19 July 2017 | | None | | |
| US | 11148279 | B1 | 19 October 2021 | US | 2022-032447 | A1 | 03 February 2022 |
| | | | | WO | 2021-247311 | A1 | 09 December 2021 |
| KR | 10-2018-0136656 | A | 26 December 2018 | US | 11109778 | B2 | 07 September 2021 |
| | | | | US | 2018-0360347 | A1 | 20 December 2018 |
| | | | | US | 2019-0046078 | A1 | 14 February 2019 |
| | | | | US | 2021-0353177 | A1 | 18 November 2021 |

Form PCT/ISA/210 (patent family annex) (July 2022)